# EUROPEAN PATENT APPLICATION

(11) **EP 1 327 684 A1**
(43) Date of publication of application: **16.07.2003**
(21) Application number: 02000820.7
(22) Date of filing: 14.01.2002
(51) Int. Cl.: C12N 15/12, C12N 15/63, C07K 14/47, C07K 16/18, A61K 38/17, A61K 31/713

(54) **DNAH5 and its use in diagnosis**

(71) Applicant: Universitätsklinikum Freiburg, 79106 Freiburg (DE)
(72) Inventor: Omran, Heymut, Dr., 79102 Freiburg (DE)
(74) Representative: Keller, Günter, Dr.

(57) **Abstract**

DNA comprising the nucleotide sequence encoding DNAH5, polypeptides comprising the amino acid sequence of DNAH5, and antibodies against DNAH5, methods for the diagnosis of diseases related to a defect of the DNAH5 gene as well as uses of the DNA and the polypeptides are described.

SEQUENCE LISTING

<110> Universitätsklinikum Freiburg

<120> DNAH5 and its use in diagnosis

<130> 20011221

<140>
<141>

<160> 6

<170> PatentIn Ver. 2.1

<210> 1
<211> 13971
<212> DNA
<213> Homo sapiens

<400> 1
attccggaag agctgactcc agcctgtcca tgcacggcta caatgtttag gattgggagg 60
agacagctct ggaagcatag cgtcactcga gttttaacgc aaagactgaa gggagagaag 120
gaagccaagc gggctctttt ggatgcgagg cataactact tatttgcaat tgtggcttcc 180
tgtttggacc tgaacaaaac cgaagtggag gatgccattc ttgaagggaa tcagattgaa 240
agaattgatc aactttttgc tgttggaggt ctccgacacc tcatgtttta ctatcaagat 300
gtggaggaag cagaaacagg acaacttggc tctctaggag gggtaaatct tgtttctgga 360
aagattaaaa aacctaaggt gttcgtgacc gagggaaacg atgtggctct tactggggta 420
tgtgtgttct tcatcaggac tgacccttcc aaagccatca cccctgacaa catccaccag 480
gaggtgagtt ttaacatgtt agatgcggca gatggaggcc tgctcaacag tgtgagacgt 540
ttgctgtcgg acatcttcat tcctgctctc agagccacga gccatggctg gggcgagctc 600
gagggccttc aggacgcagc taacattcgc caggagttct tgagctccct ggaaggcttt 660
gtgaacgtcc tgtcgggtgc acaggagagt ctgaaggaga aggtgaacct tcgaaagtgt 720
gacatacttg aactgaaaac cctaaaggaa cctacggact acttgactct agcaaataac 780
cctgagactt tgggaaaaat agaggattgc atgaaagtat ggatcaaaca gacagaacag 840
gttcttgctg aaaacaatca gctgctgaag gaagcggatg acgttgggcc acgagcggag 900
ctggagcact ggaaaaaaag actctccaag tttaactacc ttttggaaca attgaaaagc 960
ccggatgtga aggctgtgct ggcagtgctt gcggcggcca agtcgaaact gctgaagact 1020
tggcgggaga tggatattcg aatcactgat gcaactaatg aagcaaagga caatgtgaaa 1080
tacttgtata cacttgaaaa atgttgtgac cctttgtaca gcagtgatcc cctatccatg 1140
atggatgcta ttcctacact tataaatgca attaaaatga tctatagtat ctctcattac 1200
tataatacct ctgagaagat cacatctctg tttgtaaagg tgacaaatca gattatatct 1260
gcatgtaaag cctatattac caataatgga accgcttcca tctggaacca gccacaggat 1320
gttgttgaag aaaaaatact atctgcgatt aaactgaaac aggaatacca gctctgcttt 1380
cacaagacaa aacaaaagct taaacaaaat ccaaatgcaa aacaatttga ttttagcgag 1440
atgtatattt ttggaaaatt cgaaactttt caccgacgcc ttgccaagat aatagacatc 1500
tttacaaccc tcaagacgta ttcagtcctg caagattcca caattgaagg gctggaagac 1560
atggccacta aataccaggg cattgtggca accataaaga aaaaggaata caatttccta 1620

gaccagcgga aaatggattt tgaccaagat tacgaagagt tttgcaagca gactaatgac 1680
cttcataacg agttgcggaa gttcatggat gttacatttg caaagattca aaacacaaat 1740
caagctctaa gaatgttgaa gaaatttgaa agattgaata tacctaatct tggtattgat 1800
gacaaatatc aacttatcct tgagaactat ggggctgaca ttgatatgat ttcaaagctg 1860
tatacaaagc agaaatacga tcctcctctg gctcgaaacc agcctcccat cgctggaaag 1920
attttgtggg cccgccagct cttccatagg attcagcagc ccatgcagct tttccagcag 1980
cacccagctg tgctaagcac ggcagaagcc aaacctataa ttcgcagtta caacaggatg 2040
gccaaggtcc tcctggagtt tgaggtcctc ttccacaggg cgtggcttcg gcaaattgaa 2100
gaaattcatg taggtcttga ggcttcatta ttggtgaagg ctccaggcac aggggaattg 2160
tttgtaaact ttgaccctca gatattaatc ttatttagag aaacagagtg catggcccag 2220
atgggtctgg aagtctctcc actggcaact tccctcttcc agaaacgaga tagatacaaa 2280
aggaacttca gtaacatgaa gatgatgcta gctgaatatc agagagtgaa gtcaaaaata 2340
cctgctgcca ttgagcaatt gattgtccct cacttggcca aagtggatga agctctccaa 2400
cctggcttgg ctgcactgac ctggacatca ctgaatattg aggcttattt agaaaacact 2460
tttgcaaaga tcaaggacct ggagttgctg cttgacaggg tcaatgattt gattgagttc 2520
cgcattgatg ccattctaga agaaatgagc agcacgcctc tttgtcagct tccccaggag 2580
gagccactaa cctgtgaaga gtttctccaa atgacaaagg atctttgtgt aaatggtgca 2640
caaatactac attttaaaag ctcattagtg gaggaggcag tcaatgagct tgtaaatatg 2700
ttgctggatg tggaagtttt atctgaagaa gaaagtgaaa aaatatccaa tgagaatagt 2760
gttaattaca aaaatgaaag ttcagcaaaa agagaagaag gaaattttga caccttgaca 2820
tcatctatta atgccagggc caatgccctg cttttgacga cagtcacgag gaaaaagaaa 2880
gaaactgaga tgttagggga agaagcccgc gagttactct ctcatttcaa ccatcagaac 2940
atggatgctc ttctgaaagt tacaaggaat acactagagg ccattcgcaa acgtattcat 3000
tcctctcaca caattaactt ccgggacagt aacagtgcct ctaacatgaa gcagaacagt 3060
ttgcccattt tccgggcaag cgtcactctg gccattccca acatcgtcat ggcccctgcc 3120
ctggaagatg tacagcagac cctgaacaaa gccgtggagt gcatcatcag tgtccctaag 3180
ggggtcagac agtggagcag tgaactgttg tccaagaaaa agatacaaga aagaaaaatg 3240
gctgctttgc agagtaatga agacagtgat tctgatgttg aaatggagga aaatgaactt 3300
caagatacct tggagatagc atctgtaaat ttacccattc ccgtgcaaac caagaactat 3360
tataagaatg tttctgaaaa caaagagatt gtaaaattag tttctgtgct tagcacaatt 3420
atcaactcca ccaaaaagga agttattaca tccatggatt gcttcaaacg ctacaatcac 3480
atttggcaaa agggaaaaga agaagccatt aagacattta ttacacagag ccccttgctt 3540
tctgaatttg agtcccagat tctctatttc caaaacctag agcaggaaat taatgctgag 3600
cctgaatatg tctgtgtggg ttccattgct ctgtacacag ctgacttgaa gttcgccctg 3660
actgctgaga caaaggcctg gatggttgtc attggacgcc actgtaacaa aaaataccgg 3720
agtgagatgg aaaacatttt tatgcttatt gaagaattca ataagaaact aaatcgtcca 3780
attaaggacc tagatgatat tcggattgca atggcagcgc tgaaagaaat aagggaggag 3840
caaatctcca ttgactttca agtaggacct attgaggaat cttatgccct gcttaacaga 3900
tatggacttc tgatagcaag ggaagagata gacaaagttg atacactgca ctatgcttgg 3960
gagaagctgc tggcacgtgc tggcgaagtc cagaataaat tagtctcact gcagcccagt 4020
ttcaagaaag agcttattag tgctgtggag gtattcctcc aagattgtca ccagttttat 4080
ctggactatg atttgaatgg tccaatggct agcggcttga agccccagga agccagtgac 4140
aggcttatca tgtttcagaa tcaatttgat aatatctatc ggaaatacat cacatatact 4200
ggaggagagg agcttttggg cctgccagct acacagtatc ctcagcttct tgaaataaag 4260
aagcaactaa atcttctaca gaaaatatat actctgtaca acagtgtcat agaaactgta 4320
aatagctatt atgatattct ttggtcagag gtgaatattg aaaaaattaa caatgaactc 4380
ttagaattcc agaacagatg tcgaaagctt ccccgggcct tgaaggactg gcaggctttt 4440
ttggacctga agaagatcat tgatgatttc agcgagtgtt gcccgctgct ggaatacatg 4500

gccagtaaag ccatgatgga gcggcactgg gaaaggataa ccaccctcac cgggcacagt 4560
ctggatgtgg ggaatgaaag ctttaagtta agaaatatca tggaggcacc tcttctgaaa 4620
tataaagagg aaatagagga catctgtatc agtgcggtga aagagagaga cattgagcaa 4680
aagctgaagc aagtgattaa tgaatgggac aataaaacat tcaccttcgg cagctttaaa 4740
acccgtggag agctcctctt gagaggagac agtacctcgg aaatcatcgc caacatggag 4800
gacagcttga tgttgctggg atccctactg agcaacaggt acaatatgcc attcaaagcc 4860
cagattcaaa aatgggtgca gtacctttcc aactcaacag acatcatcga gagctggatg 4920
acggtgcaaa acctgtggat ttatttagaa gctgtctttg tgggaggaga cattgccaag 4980
cagctgccca aggaagccaa gcggttttct aacatagata aatcttgggt gaagatcatg 5040
actcgggcac atgaagtgcc cagtgtagtc cagtgctgtg ttggagatga gaccctgggg 5100
cagctgttac cacacttgct ggaccagttg gaaatatgcc agaaatccct tactgggtac 5160
ttggagaaaa aacgactgtg ctttcctcgg tttttcttcg tctcagatcc tgcccttcta 5220
gagattctgg ggcaggcgtc ggactcccac actatacagg cccatttgct gaatgtgttt 5280
gacaacatta aatctgtcaa gttccacgaa aagatctatg atcgaattct gtcaatttcc 5340
tctcaagagg gtgagacgat tgaattggat aaacctgtca tggcagaggg caatgtggaa 5400
gtttggctta attctctttt ggaagaatct cagtcctcat tgcatcttgt gattcgccag 5460
gcagccgcaa atattcaaga aacaggtttc caactaactg aatttctttc atccttccct 5520
gctcaggttg gattattagg aattcagatg atatggacac gggattcaga agaagccctt 5580
agaaatgcca agtttgataa aaaaatcatg cagaaaacta atcaggcttt cctggagcta 5640
ctcaatacat tgatagacgt caccacgagg gatctgagtt ccacggaacg agtgaaatac 5700
gagactctga ttactattca tgtgcaccaa agggatatct ttgatgacct gtgtcatatg 5760
catatcaaga gtcccatgga ctttgagtgg ctgaaacagt gcagatttta ctttaacgaa 5820
gattctgaca agatgatgat tcacatcaca gatgtggcgt tcatatacca gaatgaattt 5880
ttaggctgca ctgacaggct tgtaataact ccacttacag acagatgtta catcacgctg 5940
gctcaagctc tgggaatgag catgggggga gcccctgctg gacctgcagg cacaggcaaa 6000
acagaaacca ctaaagacat gggacgatgc ctcgggaaat acgtcgtggt tttcaattgt 6060
tcagaccaga tggatttccg aggacttgga cggattttta agggactggc acagtctgga 6120
tcctggggtt gttttgatga atttaaccgt attgatctac cagttctctc ggttgcagcc 6180
cagcaaattt ccattattct gacatgtaaa aaggagcaca aaaagtcttt tatctttact 6240
gatggagata atgtgactat gaaccctgaa tttgggcttt tcttaaccat gaatcctggc 6300
tatgccggac ggcaggaact ccctgaaaac ttgaagatta atttccgctc agtggccatg 6360
atggtgcctg accgtcagat tatcataagg gtgaagttgg ctagttgtgg cttcattgac 6420
aacgttgttt tggccaggaa gtttttcacg ctctacaaac tgtgtgagga gcagctttct 6480
aagcaggttc attatgactt tggcctgcgt aacattctgt cagttcttcg gaccttggga 6540
gcagcaaaaa gagccaatcc aatggatacg gagtccacga ttgtcatgcg tgtactacgg 6600
gacatgaatc tttctaaact gattgatgag gatgaaccct tgtttttgag tttgattgaa 6660
gatctctttc caaatattct tctggacaag gcaggttacc ctgaactgga agcagcaatt 6720
agtagacagg ttgaagaagc tggtttaatc aaccatcctc cttggaaact gaaggtcatc 6780
cagctattcg aaacgcagag agtgcgacat gggatgatga ctctggggcc cagtggggct 6840
gggaagacca cctgcatcca caccttgatg agagccatga cagattgtgg aaaaccacat 6900
cgggaaatga ggatgaatcc caaagcgatt actgccccac agatgtttgg tcggctggac 6960
gttgccacaa atgactggac tgatgggata ttttctacgc tttggaggaa aacattaaga 7020
gcaaagaaag gggaacatat ctggataatt cttgatggtc cagtagatgc catctggatt 7080
gaaaatctga attctgtttt ggatgataac aaaactctaa cccttgccaa tggtgatcgg 7140
attcccatgg ctccaaactg caagatcatt ttcgagcctc ataacattga caatgcttct 7200
cctgccaccg tctcaagaaa tggaatggtt ttcatgagct cttctatcct tgattggagt 7260
cctattcttg agggttttct taagaaacgc tcacctcaag aagcagaaat tcttcgtcag 7320
ctgtacaccg agtctttccc agacttgtat cgcttctgta tccagaactt agaatacaag 7380

atggaggtgc tggaggcctt tgtcatcaca cagagcatta acatgcttca aggcctgatt 7440
cctctgaagg agcaaggcgg ggaggtgagc caggctcacc tggggcggct gttcgtgttc 7500
gcgctgctgt ggagcgcggg ggcggcgctg gagctggacg gacggcgccg cctggagctc 7560
tggctgcgct ctcggcccac agggacgctg gagctgccgc cgccagcggg gcccggggac 7620
accgccttcg actactatgt ggcgcccgat ggtacatgga cgcactggaa cacgcgtacc 7680
caggaatacc tgtatccgtc tgataccacc ccagagtatg gttctattct ggtgccaaat 7740
gttgacaatg tgaggactga ctttctaatt caaaccattg ctaaacaggg caaggctgtg 7800
ctattaattg gtgaacaagg aacagccaaa acagtaataa ttaaaggatt tatgtcaaaa 7860
tatgatcctg aatgtcacat gatcaagagt ctgaattttt cttctgcaac caccccactg 7920
atgttccaga ggacgataga gagctatgtg gataaacgaa tgggtacaac atatggccct 7980
cctgcgggaa agaagatgac tgtttttatt gatgatgtga atatgccaat aatcaatgag 8040
tggggagatc aggttacgaa tgagatagtg cgacagctga tggaacaaaa tggattctat 8100
aatctagaga agcctgggga gttcaccagc atcgtggaca tccagttttt ggcagccatg 8160
atccatcctg gtggtggacg caatgacata ccccaaagac tcaagaggca gttctctata 8220
tttaattgca cgttgccctc tgaagcttct gtggacaaga tctttggtgt gattggggta 8280
ggccactact gtactcagag gggtttctca gaagaagtga gagattctgt gacaaaattg 8340
gtgcctctga cacgccgact atggcagatg accaagatta aaatgcttcc tacccctgca 8400
aaattccatt atgtgtttaa cctacgagat ctttctcggg tctggcaggg aatgctgaac 8460
actacttcag aggtcatcaa ggaaccaaat gatctgttaa agctgtggaa gcatgagtgt 8520
aaacgtgtta tagctgaccg tttcacagtg tccagtgatg tgacctggtt tgataaggct 8580
ttagtaagtt tggtagagga ggagtttggt gaagagaaaa aactcttggt ggattgtgga 8640
attgacacat attttgtgga tttcttgaga gatgcacctg aagctgcagg tgaaacatct 8700
gaagaggctg atgctgaaac acctaaaatt tatgagccaa ttgaatcttt tagtcaccta 8760
aaagagcgtc tgaatatgtt cctgcagctc tataatgaga gcatccgtgg cgccggcatg 8820
gacatggtgt tctttgcaga tgccatggtt cacttagtca agatctctcg tgtcattcgt 8880
actcctcagg gaaatgccct cctggtcggg gtgggcggat caggaaagca gagcctgacg 8940
aggttggctt cattcattgc tggctacgtt tccttccaga tcactctgac gagatcctac 9000
aacacatcaa atctgatgga agatctgaag gttttgtatc gaacagctgg tcagcaaggc 9060
aaaggaatca cttttatttt cacagacaat gagattaaag atgagtcatt tttggaatat 9120
atgaacaatg ttttatcatc aggtgaggtc tctaacctat ttgctcgaga tgaaattgat 9180
gaaattaata gcgacctggc atcagtcatg aaaaaagaat tccccaggtg ccttcctacc 9240
aatgagaacc tgcacgacta cttcatgagt cgggtccgac agaaccttca tattgtgctc 9300
tgcttctcgc cagtggggga gaaatttcga aacagagctt tgaagttccc tgccctaatt 9360
tcaggatgca caattgactg gttcagccga tggcccaaag atgctttagt tgctgtgtct 9420
gaacacttcc tcacttccta tgatattgac tgcagtttgg aaatcaagaa ggaggtggtc 9480
caatgcatgg gctccttcca ggatggggtg gctgagaagt gtgttgatta ttttcagaga 9540
ttccgacgtt ctacccacgt gacgcccaaa tcatacctct cctttattca gggctataag 9600
ttcatatatg gagaaaagca tgtggaggtg cggaccctgg ccaacagaat gaatactgga 9660
ttggaaaagc tcaaagaagc ttcagagtct gttgcagcct tgagtaaaga actggaagcg 9720
aaagaaaagg agctacaagt ggccaacgat aaagccgaca tggtcttaaa agaagtgaca 9780
atgaaagcac aggctgctga aaaggtcaag gctgaggtac agaaggtgaa ggacaggccc 9840
caggccattg tggacagcat ctctaaagac aaagccattg ctgaagaaaa actggaagca 9900
gcaaaaccag ctttagaaga ggcagaagct gcattgcaga ccatcaggcc ttcggacatc 9960
gccactgttc gcacgttggg ccgcccccct cacctcatca tgcggatcat ggattgcgta 10020
ctgctgctgt ttcaaaggaa agtcagtgct gtgaaaattg acctggaaaa aagctgtacc 10080
atgccctcct ggcaggaatc cttaaaattg atgactgcag ggaacttttt acagaactta 10140
cagcaattcc caaaagacac aatcaatgaa gaggtgatag aatttttgag tccttacttt 10200
gaaatgcctg actataacat cgaaactgct aaacgcgtat gtggaaatgt agctggtctt 10260

tgttcctgga cgaaagctat ggcttccttc ttttctataa acaaagaagt actgcctctg 10320
aaggccaact tggtggtgca agagaatcgc catctcctgg ccatgcagga tctgcagaaa 10380
gcccaggccg agttggatga caagcaggcg gaacttgacg tggtgcaggc tgagtatgaa 10440
caggccatga ctgaaaagca gaccttgctt gaagatgcag agcgatgcag acacaagatg 10500
cagacagctt ccacgctcat cagtggcttg gcaggtgaaa aagaaagatg gacagagcaa 10560
agccaagagt ttgctgcaca aactaaaaga cttgtagggg atgtactgtt ggctacagct 10620
tttctatctt attctggtcc atttaaccaa gagtttcgtg atcttctgtt aaatgactgg 10680
cggaaggaaa tgaaagcccg gaaaattcca tttggaaaga acctaaatct cagtgagatg 10740
ttgattgatg ctcctactat tagtgaatgg aacctccaag gtctgccaaa tgatgacttg 10800
tccattcaaa atggaattat tgtcacgaag gcatctcgtt accctttgtt aattgatcca 10860
cagactcaag gcaagatctg gattaaaaat aaagaaagcc gaaatgaact ccagatcacg 10920
tctttaaatc acaagtactt cagaaaccac ctggaagaca gcctttctct tggaaggcct 10980
ttgcttattg aagatgttgg agaggaacta gatccagcac tagataatgt tttggaaaga 11040
aacttcatta aaactgggtc tacctttaag gtgaaagttg gtgacaagga agtagatgtg 11100
ttggatggct ttagactcta cattaccacc aaattgccta acccagccta cacccctgag 11160
ataagtgccc gtacctccat cattgacttc actgtcacca tgaaaggtct agaagatcag 11220
ttactgggga gggtcattct cacagagaag caggaattgg agaaagaaag aactcatctg 11280
atggaagatg taactgcaaa caaaagaagg atgaaggaac tagaagataa cttgctttac 11340
cgcctgacaa gtacccaggg gtccctggta gaagatgaaa gtctcattgt cgtgctgagt 11400
aacacaaaaa ggacagccga ggaggtgaca cagaagctag aaatttctgc tgagacagaa 11460
gttcaaatta actcagcccg ggaggaatac agacctgtgg ctacgcgggg cagcatcctc 11520
tacttcctca ttactgagat gcgcttggtt aatgagatgt atcagacttc gcttcgccag 11580
tttctgggct tatttgacct ttccttagcc aggtctgtca agagcccgat tacaagcaag 11640
aggattgcta atatcatcga gcacatgacc tacgaggttt ataagtatgc tgcccgaggg 11700
ctgtacgagg agcacaaatt cctgttcacc ttgttgctta ccctaaagat tgacatccag 11760
aggaaccgag tcaagcatga agagtttctc actcttatta aaggaggtgc ctcattagac 11820
cttaaagctt gtcctccaaa accatcaaaa tggatcctgg acataacatg gctgaatttg 11880
gtggaactta gcaaactcag acagttttca gatgtccttg accagatatc gagaaatgag 11940
aaaatgtgga aaatttggtt tgataaggaa aacccggagg aggaacctct tccaaatgcc 12000
tatgataaat ctcttgactg cttcagacgt cttctcctta ttagatcctg gtgtcctgac 12060
agaaccatcg cccaggcccg caagtacatc gtggactcca tgggagaaaa atatgccgaa 12120
ggtgttattt tagacttgga gaagacgtgg gaggaatctg atccacggac gccactcatc 12180
tgtctcctgt ctatgggctc agaccccaca gattccatca ttgccttggg gaagagatta 12240
aaaatagaaa cccgttatgt gtccatgggc cagggccagg aagtccatgc tcggaagctc 12300
ttgcagcaga ccatggcgaa cggaggatgg gcacttctgc agaactgcca tctgggactt 12360
gatttcatgg atgagctgat ggacataatc atagaaactg agcttgtaca tgatgcgttc 12420
cgcctctgga tgaccaccga ggctcataag cagtttccca ttacactcct tcagatgtcc 12480
attaaatttg ccaacgatcc tccacaagga ctccgggcag gactgaaaag aacatatagt 12540
ggtgtcagcc aagacctgct ggacgtgagc tctgggtccc agtggaagcc catgctgtac 12600
gcagtggctt tcctgcactc cactgtccag gagaggcgca agttcggtgc cctggggtgg 12660
aatatcccct acgaatttaa ccaagcggac tttaatgcca ctgtgcagtt catccaaaac 12720
cacttggatg acatggatgt caaaaagggt gtctcctgga ccaccatccg ctacatgata 12780
ggagagattc aatatggagg cagagtcact gacgactatg ataagagatt gttgaacaca 12840
tttgctaagg tttggttcag tgaaaatatg tttggaccag atttcagttt ttaccaagga 12900
tacaatattc caaaatgcag cacagtggat aactatcttc agtatatcca gagtttgcct 12960
gcctatgaca gccctgaggt gtttgggctg caccccaatg ctgacatcac ctaccagagc 13020
aagctggcca aggacgtgct ggacaccatc ctaggcatcc aacccaagga cacctctggt 13080
ggaggggatg agacccggga ggcggtggtg gcccggctgg ctgatgatat gctggagaag 13140

ctgcccccag actatgtccc ctttgaagta aaagagaggc tgcagaagat ggggccattc 13200
cagcctatga acattttcct caggcaggaa atagacagaa tgcaaagggt actcagcctt 13260
gtccgcagca ccctcactga gctgaaactt gctattgatg gcaccatcat catgagcgaa 13320
aatctgcgag atgcattgga ttgcatgttt gatgctagaa tccctgcttg gtggaaaaaa 13380
gcttcttgga tttctagtac actgggtttc tggtttactg aacttataga aagaaacagc 13440
cagtttacct cgtgggtttt caatggccga cctcactgct tttggatgac gggttttttt 13500
aacccccagg gatttttaac tgcaatgcga caggaaataa ctcgggccaa caaaggctgg 13560
gctctggaca atatggtgct ttgcaatgaa gtcaccaaat ggatgaagga cgacatttct 13620
gcccctccca cagagggtgt ctatgtctat ggcttatatc ttgaaggtgc tggctgggac 13680
aagaggaaca tgaaactcat tgaatcaaag ccaaaagtgc tctttgagtt gatgcctgtc 13740
ataaggattt atgcagaaaa caatacttta cgagatcctc ggttttactc ctgtcccatc 13800
tataagaagc cagttcgaac ggacttgaac tacattgccg ctgtggatct caggacagcc 13860
cagacccctg aacactgggt gctccgtggg gttgcccttc tgtgtgatgt caagtaacat 13920
gtggggagtg tccccaccca atgctttgga aaatgcaaga tctaaattat t 13971

<210> 2
<211> 4624
<212> PRT
<213> Homo sapiens

<400> 2
Met Phe Arg Ile Gly Arg Arg Gln Leu Trp Lys His Ser Val Thr Arg
1               5                   10                  15

Val Leu Thr Gln Arg Leu Lys Gly Glu Lys Glu Ala Lys Arg Ala Leu
            20                  25                  30

Leu Asp Ala Arg His Asn Tyr Leu Phe Ala Ile Val Ala Ser Cys Leu
        35                  40                  45

Asp Leu Asn Lys Thr Glu Val Glu Asp Ala Ile Leu Glu Gly Asn Gln
    50                  55                  60

Ile Glu Arg Ile Asp Gln Leu Phe Ala Val Gly Gly Leu Arg His Leu
65                  70                  75                  80

Met Phe Tyr Tyr Gln Asp Val Glu Glu Ala Glu Thr Gly Gln Leu Gly
                85                  90                  95

Ser Leu Gly Gly Val Asn Leu Val Ser Gly Lys Ile Lys Lys Pro Lys
            100                 105                 110

Val Phe Val Thr Glu Gly Asn Asp Val Ala Leu Thr Gly Val Cys Val
        115                 120                 125

Phe Phe Ile Arg Thr Asp Pro Ser Lys Ala Ile Thr Pro Asp Asn Ile
    130                 135                 140

His Gln Glu Val Ser Phe Asn Met Leu Asp Ala Ala Asp Gly Gly Leu
145                 150                 155                 160

Leu Asn Ser Val Arg Arg Leu Leu Ser Asp Ile Phe Ile Pro Ala Leu
                165                 170                 175

Arg Ala Thr Ser His Gly Trp Gly Glu Leu Glu Gly Leu Gln Asp Ala
            180                 185                 190

Ala Asn Ile Arg Gln Glu Phe Leu Ser Ser Leu Glu Gly Phe Val Asn
        195                 200                 205

Val Leu Ser Gly Ala Gln Glu Ser Leu Lys Glu Lys Val Asn Leu Arg
    210                 215                 220

Lys Cys Asp Ile Leu Glu Leu Lys Thr Leu Lys Glu Pro Thr Asp Tyr
225                 230                 235                 240

Leu Thr Leu Ala Asn Asn Pro Glu Thr Leu Gly Lys Ile Glu Asp Cys
                245                 250                 255

Met Lys Val Trp Ile Lys Gln Thr Glu Gln Val Leu Ala Glu Asn Asn
            260                 265                 270

Gln Leu Leu Lys Glu Ala Asp Asp Val Gly Pro Arg Ala Glu Leu Glu
        275                 280                 285

His Trp Lys Lys Arg Leu Ser Lys Phe Asn Tyr Leu Leu Glu Gln Leu
    290                 295                 300

Lys Ser Pro Asp Val Lys Ala Val Leu Ala Val Leu Ala Ala Ala Lys
305                 310                 315                 320

Ser Lys Leu Leu Lys Thr Trp Arg Glu Met Asp Ile Arg Ile Thr Asp
                325                 330                 335

Ala Thr Asn Glu Ala Lys Asp Asn Val Lys Tyr Leu Tyr Thr Leu Glu
            340                 345                 350

Lys Cys Cys Asp Pro Leu Tyr Ser Ser Asp Pro Leu Ser Met Met Asp
        355                 360                 365

Ala Ile Pro Thr Leu Ile Asn Ala Ile Lys Met Ile Tyr Ser Ile Ser
    370                 375                 380

His Tyr Tyr Asn Thr Ser Glu Lys Ile Thr Ser Leu Phe Val Lys Val
385                 390                 395                 400

Thr Asn Gln Ile Ile Ser Ala Cys Lys Ala Tyr Ile Thr Asn Asn Gly
                405                 410                 415

Thr Ala Ser Ile Trp Asn Gln Pro Gln Asp Val Val Glu Glu Lys Ile
            420                 425                 430

Leu Ser Ala Ile Lys Leu Lys Gln Glu Tyr Gln Leu Cys Phe His Lys
        435                 440                 445

Thr Lys Gln Lys Leu Lys Gln Asn Pro Asn Ala Lys Gln Phe Asp Phe
    450                 455                 460

Ser Glu Met Tyr Ile Phe Gly Lys Phe Glu Thr Phe His Arg Arg Leu
465                 470                 475                 480

Ala Lys Ile Ile Asp Ile Phe Thr Thr Leu Lys Thr Tyr Ser Val Leu
                485                 490                 495

Gln Asp Ser Thr Ile Glu Gly Leu Glu Asp Met Ala Thr Lys Tyr Gln
            500                 505                 510

Gly Ile Val Ala Thr Ile Lys Lys Lys Glu Tyr Asn Phe Leu Asp Gln
        515                 520                 525

Arg Lys Met Asp Phe Asp Gln Asp Tyr Glu Glu Phe Cys Lys Gln Thr
    530                 535                 540

Asn Asp Leu His Asn Glu Leu Arg Lys Phe Met Asp Val Thr Phe Ala
545                 550                 555                 560

Lys Ile Gln Asn Thr Asn Gln Ala Leu Arg Met Leu Lys Lys Phe Glu
                565                 570                 575

Arg Leu Asn Ile Pro Asn Leu Gly Ile Asp Asp Lys Tyr Gln Leu Ile
            580                 585                 590

Leu Glu Asn Tyr Gly Ala Asp Ile Asp Met Ile Ser Lys Leu Tyr Thr
        595                 600                 605

Lys Gln Lys Tyr Asp Pro Pro Leu Ala Arg Asn Gln Pro Pro Ile Ala
    610                 615                 620

Gly Lys Ile Leu Trp Ala Arg Gln Leu Phe His Arg Ile Gln Gln Pro
625                 630                 635                 640

Met Gln Leu Phe Gln Gln His Pro Ala Val Leu Ser Thr Ala Glu Ala
                645                 650                 655

Lys Pro Ile Ile Arg Ser Tyr Asn Arg Met Ala Lys Val Leu Leu Glu
            660                 665                 670

Phe Glu Val Leu Phe His Arg Ala Trp Leu Arg Gln Ile Glu Glu Ile
        675                 680                 685

His Val Gly Leu Glu Ala Ser Leu Leu Val Lys Ala Pro Gly Thr Gly
    690                 695                 700

Glu Leu Phe Val Asn Phe Asp Pro Gln Ile Leu Ile Leu Phe Arg Glu
705                 710                 715                 720

Thr Glu Cys Met Ala Gln Met Gly Leu Glu Val Ser Pro Leu Ala Thr
                725                 730                 735

Ser Leu Phe Gln Lys Arg Asp Arg Tyr Lys Arg Asn Phe Ser Asn Met
            740                 745                 750

Lys Met Met Leu Ala Glu Tyr Gln Arg Val Lys Ser Lys Ile Pro Ala
        755                 760                 765

Ala Ile Glu Gln Leu Ile Val Pro His Leu Ala Lys Val Asp Glu Ala
    770                 775                 780

Leu Gln Pro Gly Leu Ala Ala Leu Thr Trp Thr Ser Leu Asn Ile Glu
785                 790                 795                 800

Ala Tyr Leu Glu Asn Thr Phe Ala Lys Ile Lys Asp Leu Glu Leu Leu
                805                 810                 815

Leu Asp Arg Val Asn Asp Leu Ile Glu Phe Arg Ile Asp Ala Ile Leu
            820                 825                 830

Glu Glu Met Ser Ser Thr Pro Leu Cys Gln Leu Pro Gln Glu Glu Pro
        835                 840                 845

Leu Thr Cys Glu Glu Phe Leu Gln Met Thr Lys Asp Leu Cys Val Asn
    850                 855                 860

Gly Ala Gln Ile Leu His Phe Lys Ser Ser Leu Val Glu Glu Ala Val
865                 870                 875                 880

Asn Glu Leu Val Asn Met Leu Leu Asp Val Glu Val Leu Ser Glu Glu
                885                 890                 895

Glu Ser Glu Lys Ile Ser Asn Glu Asn Ser Val Asn Tyr Lys Asn Glu
            900                 905                 910

Ser Ser Ala Lys Arg Glu Glu Gly Asn Phe Asp Thr Leu Thr Ser Ser
        915                 920                 925

Ile Asn Ala Arg Ala Asn Ala Leu Leu Leu Thr Thr Val Thr Arg Lys
    930                 935                 940

Lys Lys Glu Thr Glu Met Leu Gly Glu Glu Ala Arg Glu Leu Leu Ser
945                 950                 955                 960

His Phe Asn His Gln Asn Met Asp Ala Leu Leu Lys Val Thr Arg Asn
                965                 970                 975

Thr Leu Glu Ala Ile Arg Lys Arg Ile His Ser Ser His Thr Ile Asn
            980                 985                 990

Phe Arg Asp Ser Asn Ser Ala Ser Asn Met Lys Gln Asn Ser Leu Pro
        995                 1000                1005

Ile Phe Arg Ala Ser Val Thr Leu Ala Ile Pro Asn Ile Val Met Ala
    1010                1015                1020

Pro Ala Leu Glu Asp Val Gln Gln Thr Leu Asn Lys Ala Val Glu Cys
1025                1030                1035                1040

Ile Ile Ser Val Pro Lys Gly Val Arg Gln Trp Ser Ser Glu Leu Leu
                1045                1050                1055

Ser Lys Lys Lys Ile Gln Glu Arg Lys Met Ala Ala Leu Gln Ser Asn
            1060                1065                1070

Glu Asp Ser Asp Ser Asp Val Glu Met Gly Glu Asn Glu Leu Gln Asp
        1075                1080                1085

Thr Leu Glu Ile Ala Ser Val Asn Leu Pro Ile Pro Val Gln Thr Lys
    1090                1095                1100

Asn Tyr Tyr Lys Asn Val Ser Glu Asn Lys Glu Ile Val Lys Leu Val
1105                1110                1115                1120

Ser Val Leu Ser Thr Ile Ile Asn Ser Thr Lys Lys Glu Val Ile Thr
                1125                1130                1135

Ser Met Asp Cys Phe Lys Arg Tyr Asn His Ile Trp Gln Lys Gly Lys
            1140                1145                1150

Glu Glu Ala Ile Lys Thr Phe Ile Thr Gln Ser Pro Leu Leu Ser Glu
        1155                1160                1165

Phe Glu Ser Gln Ile Leu Tyr Phe Gln Asn Leu Glu Gln Glu Ile Asn
    1170                1175                1180

Ala Glu Pro Glu Tyr Val Cys Val Gly Ser Ile Ala Leu Tyr Thr Ala
1185                1190                1195                1200

Asp Leu Lys Phe Ala Leu Thr Ala Glu Thr Lys Ala Trp Met Val Val
                1205                1210                1215

Ile Gly Arg His Cys Asn Lys Lys Tyr Arg Ser Glu Met Glu Asn Ile
            1220                1225                1230

Phe Met Leu Ile Glu Glu Phe Asn Lys Lys Leu Asn Arg Pro Ile Lys
        1235                1240                1245

Asp Leu Asp Asp Ile Arg Ile Ala Met Ala Ala Leu Lys Glu Ile Arg
    1250                1255                1260

Glu Glu Gln Ile Ser Ile Asp Phe Gln Val Gly Pro Ile Glu Glu Ser
1265                1270                1275                1280

Tyr Ala Leu Leu Asn Arg Tyr Gly Leu Leu Ile Ala Arg Glu Glu Ile
                1285                1290                1295

Asp Lys Val Asp Thr Leu His Tyr Ala Trp Glu Lys Leu Leu Ala Arg
            1300                1305                1310

Ala Gly Glu Val Gln Asn Lys Leu Val Ser Leu Gln Pro Ser Phe Lys
        1315                1320                1325

Lys Glu Leu Ile Ser Ala Val Glu Val Phe Leu Gln Asp Cys His Gln
    1330                1335                1340

Phe Tyr Leu Asp Tyr Asp Leu Asn Gly Pro Met Ala Ser Gly Leu Lys
1345                1350                1355                1360

Pro Gln Glu Ala Ser Asp Arg Leu Ile Met Phe Gln Asn Gln Phe Asp
                1365                1370                1375

Asn Ile Tyr Arg Lys Tyr Ile Thr Tyr Thr Gly Gly Glu Glu Leu Phe
            1380                1385                1390

Gly Leu Pro Ala Thr Gln Tyr Pro Gln Leu Leu Glu Ile Lys Lys Gln
        1395                1400                1405

Leu Asn Leu Leu Gln Lys Ile Tyr Thr Leu Tyr Asn Ser Val Ile Glu
    1410                1415                1420

Thr Val Asn Ser Tyr Tyr Asp Ile Leu Trp Ser Glu Val Asn Ile Glu
1425                1430                1435                1440

Lys Ile Asn Asn Glu Leu Leu Glu Phe Gln Asn Arg Cys Arg Lys Leu
                1445                1450                1455

Pro Arg Ala Leu Lys Asp Trp Gln Ala Phe Leu Asp Leu Lys Lys Ile
            1460                1465                1470

Ile Asp Asp Phe Ser Glu Cys Cys Pro Leu Leu Glu Tyr Met Ala Ser
        1475                1480                1485

Lys Ala Met Met Glu Arg His Trp Glu Arg Ile Thr Thr Leu Thr Gly
    1490                1495                1500

His Ser Leu Asp Val Gly Asn Glu Ser Phe Lys Leu Arg Asn Ile Met
1505                1510                1515                1520

Glu Ala Pro Leu Leu Lys Tyr Lys Glu Glu Ile Glu Asp Ile Cys Ile
                1525                1530                1535

Ser Ala Val Lys Glu Arg Asp Ile Glu Gln Lys Leu Lys Gln Val Ile
            1540                1545                1550

Asn Glu Trp Asp Asn Lys Thr Phe Thr Phe Gly Ser Phe Lys Thr Arg
        1555                1560                1565

Gly Glu Leu Leu Leu Arg Gly Asp Ser Thr Ser Glu Ile Ile Ala Asn
    1570                1575                1580

Met Glu Asp Ser Leu Met Leu Leu Gly Ser Leu Leu Ser Asn Arg Tyr
1585                1590                1595                1600

Asn Met Pro Phe Lys Ala Gln Ile Gln Lys Trp Val Gln Tyr Leu Ser
                1605                1610                1615

Asn Ser Thr Asp Ile Ile Glu Ser Trp Met Thr Val Gln Asn Leu Trp
            1620                1625                1630

Ile Tyr Leu Glu Ala Val Phe Val Gly Gly Asp Ile Ala Lys Gln Leu
        1635                1640                1645

Pro Lys Glu Ala Lys Arg Phe Ser Asn Ile Asp Lys Ser Trp Val Lys
    1650                1655                1660

Ile Met Thr Arg Ala His Glu Val Pro Ser Val Val Gln Cys Cys Val
1665                1670                1675                1680

Gly Asp Glu Thr Leu Gly Gln Leu Leu Pro His Leu Leu Asp Gln Leu
            1685                1690                1695

Glu Ile Cys Gln Lys Ser Leu Thr Gly Tyr Leu Glu Lys Lys Arg Leu
        1700                1705                1710

Cys Phe Pro Arg Phe Phe Phe Val Ser Asp Pro Ala Leu Leu Glu Ile
    1715                1720                1725

Leu Gly Gln Ala Ser Asp Ser His Thr Ile Gln Ala His Leu Leu Asn
1730                1735                1740

Val Phe Asp Asn Ile Lys Ser Val Lys Phe His Glu Lys Ile Tyr Asp
1745                1750                1755                1760

Arg Ile Leu Ser Ile Ser Ser Gln Glu Gly Glu Thr Ile Glu Leu Asp
            1765                1770                1775

Lys Pro Val Met Ala Glu Gly Asn Val Glu Val Trp Leu Asn Ser Leu
        1780                1785                1790

Leu Glu Glu Ser Gln Ser Ser Leu His Leu Val Ile Arg Gln Ala Ala
    1795                1800                1805

Ala Asn Ile Gln Glu Thr Gly Phe Gln Leu Thr Glu Phe Leu Ser Ser
1810                1815                1820

Phe Pro Ala Gln Val Gly Leu Leu Gly Ile Gln Met Ile Trp Thr Arg
1825                1830                1835                1840

Asp Ser Glu Glu Ala Leu Arg Asn Ala Lys Phe Asp Lys Lys Ile Met
            1845                1850                1855

Gln Lys Thr Asn Gln Ala Phe Leu Glu Leu Leu Asn Thr Leu Ile Asp
        1860                1865                1870

Val Thr Thr Arg Asp Leu Ser Ser Thr Glu Arg Val Lys Tyr Glu Thr
    1875                1880                1885

Leu Ile Thr Ile His Val His Gln Arg Asp Ile Phe Asp Asp Leu Cys
1890                1895                1900

His Met His Ile Lys Ser Pro Met Asp Phe Glu Trp Leu Lys Gln Cys
1905                1910                1915                1920

Arg Phe Tyr Phe Asn Glu Asp Ser Asp Lys Met Met Ile His Ile Thr
            1925                1930                1935

Asp Val Ala Phe Ile Tyr Gln Asn Glu Phe Leu Gly Cys Thr Asp Arg
            1940                1945                1950

Leu Val Ile Thr Pro Leu Thr Asp Arg Cys Tyr Ile Thr Leu Ala Gln
        1955                1960                1965

Ala Leu Gly Met Ser Met Gly Gly Ala Pro Ala Gly Pro Ala Gly Thr
    1970                1975                1980

Gly Lys Thr Glu Thr Thr Lys Asp Met Gly Arg Cys Leu Gly Lys Tyr
1985                1990                1995                2000

Val Val Val Phe Asn Cys Ser Asp Gln Met Asp Phe Arg Gly Leu Gly
                2005                2010                2015

Arg Ile Phe Lys Gly Leu Ala Gln Ser Gly Ser Trp Gly Cys Phe Asp
            2020                2025                2030

Glu Phe Asn Arg Ile Asp Leu Pro Val Leu Ser Val Ala Ala Gln Gln
        2035                2040                2045

Ile Ser Ile Ile Leu Thr Cys Lys Lys Glu His Lys Lys Ser Phe Ile
    2050                2055                2060

Phe Thr Asp Gly Asp Asn Val Thr Met Asn Pro Glu Phe Gly Leu Phe
2065                2070                2075                2080

Leu Thr Met Asn Pro Gly Tyr Ala Gly Arg Gln Glu Leu Pro Glu Asn
                2085                2090                2095

Leu Lys Ile Asn Phe Arg Ser Val Ala Met Met Val Pro Asp Arg Gln
            2100                2105                2110

Ile Ile Ile Arg Val Lys Leu Ala Ser Cys Gly Phe Ile Asp Asn Val
        2115                2120                2125

Val Leu Ala Arg Lys Phe Phe Thr Leu Tyr Lys Leu Cys Glu Glu Gln
    2130                2135                2140

Leu Ser Lys Gln Val His Tyr Asp Phe Gly Leu Arg Asn Ile Leu Ser
2145                2150                2155                2160

Val Leu Arg Thr Leu Gly Ala Ala Lys Arg Ala Asn Pro Met Asp Thr
                2165                2170                2175

Glu Ser Thr Ile Val Met Arg Val Leu Arg Asp Met Asn Leu Ser Lys
            2180                2185                2190

Leu Ile Asp Glu Asp Glu Pro Leu Phe Leu Ser Leu Ile Glu Asp Leu
        2195                2200                2205

Phe Pro Asn Ile Leu Leu Asp Lys Ala Gly Tyr Pro Glu Leu Glu Ala
    2210                2215                2220

Ala Ile Ser Arg Gln Val Glu Glu Ala Gly Leu Ile Asn His Pro Pro
2225                2230                2235                2240

Trp Lys Leu Lys Val Ile Gln Leu Phe Glu Thr Gln Arg Val Arg His
                2245                2250                2255

Gly Met Met Thr Leu Gly Pro Ser Gly Ala Gly Lys Thr Thr Cys Ile
            2260                2265                2270

His Thr Leu Met Arg Ala Met Thr Asp Cys Gly Lys Pro His Arg Glu
        2275                2280                2285

Met Arg Met Asn Pro Lys Ala Ile Thr Ala Pro Gln Met Phe Gly Arg
    2290                2295                2300

Leu Asp Val Ala Thr Asn Asp Trp Thr Asp Gly Ile Phe Ser Thr Leu
2305                2310                2315                2320

Trp Arg Lys Thr Leu Arg Ala Lys Lys Gly Glu His Ile Trp Ile Ile
                2325                2330                2335

Leu Asp Gly Pro Val Asp Ala Ile Trp Ile Glu Asn Leu Asn Ser Val
            2340                2345                2350

Leu Asp Asp Asn Lys Thr Leu Thr Leu Ala Asn Gly Asp Arg Ile Pro
        2355                2360                2365

Met Ala Pro Asn Cys Lys Ile Ile Phe Glu Pro His Asn Ile Asp Asn
    2370                2375                2380

Ala Ser Pro Ala Thr Val Ser Arg Asn Gly Met Val Phe Met Ser Ser
2385                2390                2395                2400

Ser Ile Leu Asp Trp Ser Pro Ile Leu Glu Gly Phe Leu Lys Lys Arg
                2405                2410                2415

Ser Pro Gln Glu Ala Glu Ile Leu Arg Gln Leu Tyr Thr Glu Ser Phe
            2420                2425                2430

Pro Asp Leu Tyr Arg Phe Cys Ile Gln Asn Leu Glu Tyr Lys Met Glu
        2435                2440                2445

Val Leu Glu Ala Phe Val Ile Thr Gln Ser Ile Asn Met Leu Gln Gly
    2450                2455                2460

Leu Ile Pro Leu Lys Glu Gln Gly Gly Glu Val Ser Gln Ala His Leu
2465                2470                2475                2480

Gly Arg Leu Phe Val Phe Ala Leu Leu Trp Ser Ala Gly Ala Ala Leu
                2485                2490                2495

Glu Leu Asp Gly Arg Arg Arg Leu Glu Leu Trp Leu Arg Ser Arg Pro
            2500                2505                2510

Thr Gly Thr Leu Glu Leu Pro Pro Pro Ala Gly Pro Gly Asp Thr Ala
        2515                2520                2525

Phe Asp Tyr Tyr Val Ala Pro Asp Gly Thr Trp Thr His Trp Asn Thr
    2530                2535                2540

Arg Thr Gln Glu Tyr Leu Tyr Pro Ser Asp Thr Thr Pro Glu Tyr Gly
2545                2550                2555                2560

Ser Ile Leu Val Pro Asn Val Asp Asn Val Arg Thr Asp Phe Leu Ile
                2565                2570                2575

Gln Thr Ile Ala Lys Gln Gly Lys Ala Val Leu Leu Ile Gly Glu Gln
            2580                2585                2590

Gly Thr Ala Lys Thr Val Ile Ile Lys Gly Phe Met Ser Lys Tyr Asp
        2595                2600                2605

Pro Glu Cys His Met Ile Lys Ser Leu Asn Phe Ser Ser Ala Thr Thr
    2610                2615                2620

Pro Leu Met Phe Gln Arg Thr Ile Glu Ser Tyr Val Asp Lys Arg Met
2625                2630                2635                2640

Gly Thr Thr Tyr Gly Pro Pro Ala Gly Lys Lys Met Thr Val Phe Ile
                2645                2650                2655

Asp Asp Val Asn Met Pro Ile Ile Asn Glu Trp Gly Asp Gln Val Thr
            2660                2665                2670

Asn Glu Ile Val Arg Gln Leu Met Glu Gln Asn Gly Phe Tyr Asn Leu
        2675                2680                2685

Glu Lys Pro Gly Glu Phe Thr Ser Ile Val Asp Ile Gln Phe Leu Ala
    2690                2695                2700

Ala Met Ile His Pro Gly Gly Gly Arg Asn Asp Ile Pro Gln Arg Leu
2705                2710                2715                2720

Lys Arg Gln Phe Ser Ile Phe Asn Cys Thr Leu Pro Ser Glu Ala Ser
                2725                2730                2735

Val Asp Lys Ile Phe Gly Val Ile Gly Val Gly His Tyr Cys Thr Gln
            2740                2745                2750

Arg Gly Phe Ser Glu Glu Val Arg Asp Ser Val Thr Lys Leu Val Pro
        2755                2760                2765

Leu Thr Arg Arg Leu Trp Gln Met Thr Lys Ile Lys Met Leu Pro Thr
    2770                2775                2780

Pro Ala Lys Phe His Tyr Val Phe Asn Leu Arg Asp Leu Ser Arg Val
2785                2790                2795                2800

Trp Gln Gly Met Leu Asn Thr Thr Ser Glu Val Ile Lys Glu Pro Asn
                2805                2810                2815

Asp Leu Leu Lys Leu Trp Lys His Glu Cys Lys Arg Val Ile Ala Asp
            2820                2825                2830

Arg Phe Thr Val Ser Ser Asp Val Thr Trp Phe Asp Lys Ala Leu Val
        2835                2840                2845

Ser Leu Val Glu Glu Glu Phe Gly Glu Glu Lys Lys Leu Leu Val Asp
    2850                2855                2860

Cys Gly Ile Asp Thr Tyr Phe Val Asp Phe Leu Arg Asp Ala Pro Glu
2865                2870                2875                2880

Ala Ala Gly Glu Thr Ser Glu Glu Ala Asp Ala Glu Thr Pro Lys Ile
                2885                2890                2895

Tyr Glu Pro Ile Glu Ser Phe Ser His Leu Lys Glu Arg Leu Asn Met
            2900                2905                2910

Phe Leu Gln Leu Tyr Asn Glu Ser Ile Arg Gly Ala Gly Met Asp Met
        2915                2920                2925

Val Phe Phe Ala Asp Ala Met Val His Leu Val Lys Ile Ser Arg Val
    2930                2935                2940

Ile Arg Thr Pro Gln Gly Asn Ala Leu Leu Val Gly Val Gly Gly Ser
2945                2950                2955                2960

Gly Lys Gln Ser Leu Thr Arg Leu Ala Ser Phe Ile Ala Gly Tyr Val
                2965                2970                2975

Ser Phe Gln Ile Thr Leu Thr Arg Ser Tyr Asn Thr Ser Asn Leu Met
            2980                2985                2990

Glu Asp Leu Lys Val Leu Tyr Arg Thr Ala Gly Gln Gln Gly Lys Gly
        2995                3000                3005

Ile Thr Phe Ile Phe Thr Asp Asn Glu Ile Lys Asp Glu Ser Phe Leu
    3010                3015                3020

Glu Tyr Met Asn Asn Val Leu Ser Ser Gly Glu Val Ser Asn Leu Phe
3025                3030                3035                3040

Ala Arg Asp Glu Ile Asp Glu Ile Asn Ser Asp Leu Ala Ser Val Met
                3045                3050                3055

Lys Lys Glu Phe Pro Arg Cys Leu Pro Thr Asn Glu Asn Leu His Asp
            3060                3065                3070

Tyr Phe Met Ser Arg Val Arg Gln Asn Leu His Ile Val Leu Cys Phe
        3075                3080                3085

Ser Pro Val Gly Glu Lys Phe Arg Asn Arg Ala Leu Lys Phe Pro Ala
    3090                3095                3100

Leu Ile Ser Gly Cys Thr Ile Asp Trp Phe Ser Arg Trp Pro Lys Asp
3105                3110                3115                3120

Ala Leu Val Ala Val Ser Glu His Phe Leu Thr Ser Tyr Asp Ile Asp
                3125                3130                3135

Cys Ser Leu Glu Ile Lys Lys Glu Val Val Gln Cys Met Gly Ser Phe
            3140                3145                3150

Gln Asp Gly Val Ala Glu Lys Cys Val Asp Tyr Phe Gln Arg Phe Arg
        3155                3160                3165

Arg Ser Thr His Val Thr Pro Lys Ser Tyr Leu Ser Phe Ile Gln Gly
    3170                3175                3180

Tyr Lys Phe Ile Tyr Gly Glu Lys His Val Glu Val Arg Thr Leu Ala
3185                3190                3195                3200

Asn Arg Met Asn Thr Gly Leu Glu Lys Leu Lys Glu Ala Ser Glu Ser
                3205                3210                3215

Val Ala Ala Leu Ser Lys Glu Leu Glu Ala Lys Glu Lys Glu Leu Gln
            3220                3225                3230

Val Ala Asn Asp Lys Ala Asp Met Val Leu Lys Glu Val Thr Met Lys
        3235                3240                3245

Ala Gln Ala Ala Glu Lys Val Lys Ala Glu Val Gln Lys Val Lys Asp
    3250                3255                3260

Arg Ala Gln Ala Ile Val Asp Ser Ile Ser Lys Asp Lys Ala Ile Ala
3265                3270                3275                3280

Glu Glu Lys Leu Glu Ala Ala Lys Pro Ala Leu Glu Glu Ala Glu Ala
                3285                3290                3295

Ala Leu Gln Thr Ile Arg Pro Ser Asp Ile Ala Thr Val Arg Thr Leu
            3300                3305                3310

Gly Arg Pro Pro His Leu Ile Met Arg Ile Met Asp Cys Val Leu Leu
        3315                3320                3325

Leu Phe Gln Arg Lys Val Ser Ala Val Lys Ile Asp Leu Glu Lys Ser
    3330                3335                3340

Cys Thr Met Pro Ser Trp Gln Glu Ser Leu Lys Leu Met Thr Ala Gly
3345                3350                3355                3360

Asn Phe Leu Gln Asn Leu Gln Gln Phe Pro Lys Asp Thr Ile Asn Glu
                3365                3370                3375

Glu Val Ile Glu Phe Leu Ser Pro Tyr Phe Glu Met Pro Asp Tyr Asn
            3380                3385                3390

Ile Glu Thr Ala Lys Arg Val Cys Gly Asn Val Ala Gly Leu Cys Ser
        3395                3400                3405

Trp Thr Lys Ala Met Ala Ser Phe Phe Ser Ile Asn Lys Glu Val Leu
    3410                3415                3420

Pro Leu Lys Ala Asn Leu Val Val Gln Glu Asn Arg His Leu Leu Ala
3425                3430                3435                3440

Met Gln Asp Leu Gln Lys Ala Gln Ala Glu Leu Asp Asp Lys Gln Ala
                3445                3450                3455

Glu Leu Asp Val Val Gln Ala Glu Tyr Glu Gln Ala Met Thr Glu Lys
            3460                3465                3470

Gln Thr Leu Leu Glu Asp Ala Glu Arg Cys Arg His Lys Met Gln Thr
        3475                3480                3485

Ala Ser Thr Leu Ile Ser Gly Leu Ala Gly Glu Lys Glu Arg Trp Thr
    3490                3495                3500

Glu Gln Ser Gln Glu Phe Ala Ala Gln Thr Lys Arg Leu Val Gly Asp
3505                3510                3515                3520

Val Leu Leu Ala Thr Ala Phe Leu Ser Tyr Ser Gly Pro Phe Asn Gln
                3525                3530                3535

Glu Phe Arg Asp Leu Leu Leu Asn Asp Trp Arg Lys Glu Met Lys Ala
            3540                3545                3550

Arg Lys Ile Pro Phe Gly Lys Asn Leu Asn Leu Ser Glu Met Leu Ile
        3555                3560                3565

Asp Ala Pro Thr Ile Ser Glu Trp Asn Leu Gln Gly Leu Pro Asn Asp
    3570                3575                3580

Asp Leu Ser Ile Gln Asn Gly Ile Ile Val Thr Lys Ala Ser Arg Tyr
3585                3590                3595                3600

Pro Leu Leu Ile Asp Pro Gln Thr Gln Gly Lys Ile Trp Ile Lys Asn
                3605                3610                3615

Lys Glu Ser Arg Asn Glu Leu Gln Ile Thr Ser Leu Asn His Lys Tyr
            3620                3625                3630

Phe Arg Asn His Leu Glu Asp Ser Leu Ser Leu Gly Arg Pro Leu Leu
        3635                3640                3645

Ile Glu Asp Val Gly Glu Glu Leu Asp Pro Ala Leu Asp Asn Val Leu
    3650                3655                3660

Glu Arg Asn Phe Ile Lys Thr Gly Ser Thr Phe Lys Val Lys Val Gly
3665                3670                3675                3680

Asp Lys Glu Val Asp Val Leu Asp Gly Phe Arg Leu Tyr Ile Thr Thr
                3685                3690                3695

Lys Leu Pro Asn Pro Ala Tyr Thr Pro Glu Ile Ser Ala Arg Thr Ser
            3700                3705                3710

Ile Ile Asp Phe Thr Val Thr Met Lys Gly Leu Glu Asp Gln Leu Leu
        3715                3720                3725

Gly Arg Val Ile Leu Thr Glu Lys Gln Glu Leu Glu Lys Glu Arg Thr
    3730                3735                3740

His Leu Met Glu Asp Val Thr Ala Asn Lys Arg Arg Met Lys Glu Leu
3745                3750                3755                3760

Glu Asp Asn Leu Leu Tyr Arg Leu Thr Ser Thr Gln Gly Ser Leu Val
                3765                3770                3775

Glu Asp Glu Ser Leu Ile Val Val Leu Ser Asn Thr Lys Arg Thr Ala
            3780                3785                3790

Glu Glu Val Thr Gln Lys Leu Glu Ile Ser Ala Glu Thr Glu Val Gln
        3795                3800                3805

Ile Asn Ser Ala Arg Glu Glu Tyr Arg Pro Val Ala Thr Arg Gly Ser
    3810                3815                3820

Ile Leu Tyr Phe Leu Ile Thr Glu Met Arg Leu Val Asn Glu Met Tyr
3825                3830                3835                3840

Gln Thr Ser Leu Arg Gln Phe Leu Gly Leu Phe Asp Leu Ser Leu Ala
                3845                3850                3855

Arg Ser Val Lys Ser Pro Ile Thr Ser Lys Arg Ile Ala Asn Ile Ile
            3860                3865                3870

Glu His Met Thr Tyr Glu Val Tyr Lys Tyr Ala Ala Arg Gly Leu Tyr
        3875                3880                3885

Glu Glu His Lys Phe Leu Phe Thr Leu Leu Leu Thr Leu Lys Ile Asp
    3890                3895                3900

Ile Gln Arg Asn Arg Val Lys His Glu Glu Phe Leu Thr Leu Ile Lys
3905                3910                3915                3920

Gly Gly Ala Ser Leu Asp Leu Lys Ala Cys Pro Pro Lys Pro Ser Lys
                3925                3930                3935

Trp Ile Leu Asp Ile Thr Trp Leu Asn Leu Val Glu Leu Ser Lys Leu
            3940                3945                3950

Arg Gln Phe Ser Asp Val Leu Asp Gln Ile Ser Arg Asn Glu Lys Met
        3955                3960                3965

Trp Lys Ile Trp Phe Asp Lys Glu Asn Pro Glu Glu Glu Pro Leu Pro
    3970                3975                3980

Asn Ala Tyr Asp Lys Ser Leu Asp Cys Phe Arg Arg Leu Leu Leu Ile
3985                3990                3995                4000

Arg Ser Trp Cys Pro Asp Arg Thr Ile Ala Gln Ala Arg Lys Tyr Ile
                4005                4010                4015

Val Asp Ser Met Gly Glu Lys Tyr Ala Glu Gly Val Ile Leu Asp Leu
            4020                4025                4030

Glu Lys Thr Trp Glu Glu Ser Asp Pro Arg Thr Pro Leu Ile Cys Leu
        4035                4040                4045

Leu Ser Met Gly Ser Asp Pro Thr Asp Ser Ile Ile Ala Leu Gly Lys
    4050                4055                4060

Arg Leu Lys Ile Glu Thr Arg Tyr Val Ser Met Gly Gln Gly Gln Glu
4065                4070                4075                4080

Val His Ala Arg Lys Leu Leu Gln Gln Thr Met Ala Asn Gly Gly Trp
                4085                4090                4095

Ala Leu Leu Gln Asn Cys His Leu Gly Leu Asp Phe Met Asp Glu Leu
            4100                4105                4110

Met Asp Ile Ile Ile Glu Thr Glu Leu Val His Asp Ala Phe Arg Leu
        4115                4120                4125

Trp Met Thr Thr Glu Ala His Lys Gln Phe Pro Ile Thr Leu Leu Gln
    4130                4135                4140

Met Ser Ile Lys Phe Ala Asn Asp Pro Pro Gln Gly Leu Arg Ala Gly
4145                4150                4155                4160

Leu Lys Arg Thr Tyr Ser Gly Val Ser Gln Asp Leu Leu Asp Val Ser
                4165                4170                4175

Ser Gly Ser Gln Trp Lys Pro Met Leu Tyr Ala Val Ala Phe Leu His
            4180                4185                4190

Ser Thr Val Gln Glu Arg Arg Lys Phe Gly Ala Leu Gly Trp Asn Ile
        4195                4200                4205

Pro Tyr Glu Phe Asn Gln Ala Asp Phe Asn Ala Thr Val Gln Phe Ile
    4210                4215                4220

Gln Asn His Leu Asp Asp Met Asp Val Lys Lys Gly Val Ser Trp Thr
4225                4230                4235                4240

Thr Ile Arg Tyr Met Ile Gly Glu Ile Gln Tyr Gly Gly Arg Val Thr
                4245                4250                4255

Asp Asp Tyr Asp Lys Arg Leu Leu Asn Thr Phe Ala Lys Val Trp Phe
            4260                4265                4270

Ser Glu Asn Met Phe Gly Pro Asp Phe Ser Phe Tyr Gln Gly Tyr Asn
        4275                4280                4285

Ile Pro Lys Cys Ser Thr Val Asp Asn Tyr Leu Gln Tyr Ile Gln Ser
    4290                4295                4300

Leu Pro Ala Tyr Asp Ser Pro Glu Val Phe Gly Leu His Pro Asn Ala
4305                4310                4315                4320

Asp Ile Thr Tyr Gln Ser Lys Leu Ala Lys Asp Val Leu Asp Thr Ile
                4325                4330                4335

Leu Gly Ile Gln Pro Lys Asp Thr Ser Gly Gly Gly Asp Glu Thr Arg
            4340                4345                4350

Glu Ala Val Val Ala Arg Leu Ala Asp Asp Met Leu Glu Lys Leu Pro
        4355                4360                4365

Pro Asp Tyr Val Pro Phe Glu Val Lys Glu Arg Leu Gln Lys Met Gly
    4370                4375                4380

Pro Phe Gln Pro Met Asn Ile Phe Leu Arg Gln Glu Ile Asp Arg Met
4385                4390                4395                4400

Gln Arg Val Leu Ser Leu Val Arg Ser Thr Leu Thr Glu Leu Lys Leu
                4405                4410                4415

Ala Ile Asp Gly Thr Ile Ile Met Ser Glu Asn Leu Arg Asp Ala Leu
            4420                4425                4430

Asp Cys Met Phe Asp Ala Arg Ile Pro Ala Trp Trp Lys Lys Ala Ser
        4435                4440                4445

Trp Ile Ser Ser Thr Leu Gly Phe Trp Phe Thr Glu Leu Ile Glu Arg
    4450                4455                4460

Asn Ser Gln Phe Thr Ser Trp Val Phe Asn Gly Arg Pro His Cys Phe
4465                4470                4475                4480

Trp Met Thr Gly Phe Phe Asn Pro Gln Gly Phe Leu Thr Ala Met Arg
                4485                4490                4495

Gln Glu Ile Thr Arg Ala Asn Lys Gly Trp Ala Leu Asp Asn Met Val
            4500                4505                4510

Leu Cys Asn Glu Val Thr Lys Trp Met Lys Asp Asp Ile Ser Ala Pro
        4515                4520                4525

Pro Thr Glu Gly Val Tyr Val Tyr Gly Leu Tyr Leu Glu Gly Ala Gly
    4530                4535                4540

Trp Asp Lys Arg Asn Met Lys Leu Ile Glu Ser Lys Pro Lys Val Leu
4545                4550                4555                4560

Phe Glu Leu Met Pro Val Ile Arg Ile Tyr Ala Glu Asn Asn Thr Leu
                4565                4570                4575

Arg Asp Pro Arg Phe Tyr Ser Cys Pro Ile Tyr Lys Lys Pro Val Arg
            4580                4585                4590

Thr Asp Leu Asn Tyr Ile Ala Ala Val Asp Leu Arg Thr Ala Gln Thr
        4595                4600                4605

Pro Glu His Trp Val Leu Arg Gly Val Ala Leu Leu Cys Asp Val Lys
    4610                4615                4620

<210> 3
<211> 20
<212> DNA
<213> Artificial Sequence

<220>
<223> Description of Artificial Sequence: primer

<400> 3
tttcccccct tacagtatgg                                              20

<210> 4
<211> 20
<212> DNA
<213> Artificial Sequence

<220>
<223> Description of Artificial Sequence: primer

<400> 4
aacaagagcg agactccgtc                                                    20

<210> 5
<211> 20
<212> DNA
<213> Artificial Sequence

<220>
<223> Description of Artificial Sequence: primer

<400> 5
tagccaacat tgcactccag                                                    20

<210> 6
<211> 21
<212> DNA
<213> Artificial Sequence

<220>
<223> Description of Artificial Sequence: primer

<400> 6
ctgtccttga gaatcttcac a                                                  21

## Description

The present invention is concerned with a DNA comprising the nucleotide sequence encoding DNAH5, polypeptides comprising the amino acid sequence of DNAH5, and antibodies against DNAH5, methods for the diagnosis of diseases related to a defect of the DNAH5 gene as well as uses of the DNA and the polypeptides.

Dyneins are acting as molecular motors which convert the energy of ATP into force for translocation and are moving along the microtubules. Dynein is present on cilia and flagella and thus is related to the mucociliary clearance in the respiratory tract and the mobility of the sperms. They are protein complexes composed of several heavy, light, and intermediate chains. Dynein heavy chains (DHCs) are responsible for force production and ATPase activity and contain a highly conserved catalytic domain with 4 P-loop consensus motifs involed in nucleotide binding. The axonemal dynein heavy chain is referred to as DNAH5.

It has now been found by the inventors of the present application that dynein, or exactly one of the heavy chains of dynein, DNAH5, is connected with diseases like primary ciliary dyskinesia (PCD, MIM 242650). PCD is characterized by recurrent infections of the respiratory tract due to reduced mucociliary clearance, sperm immobility, and *situs inversus* in half of the affected off-spring due to randomization of left-right (LR) asymmetry (Afzelius, B.A. & Mossberg, B. The Metabolic and molecular bases of inherited disease (eds. Scriver, C.R., Beaudet, A.L. & Sly, W.S.) 3943-3954 (McGraw-Hill, New York, 1995).

It was the object of the present invention to identify the DNA sequence of the polypeptide NAH5 of the gene encoding DNAH5 as well as the amino acid sequence of the said protein.

Moreover, it was the object of the present invention to provide a method for the diagnosis of primary ciliary dyskinesia.

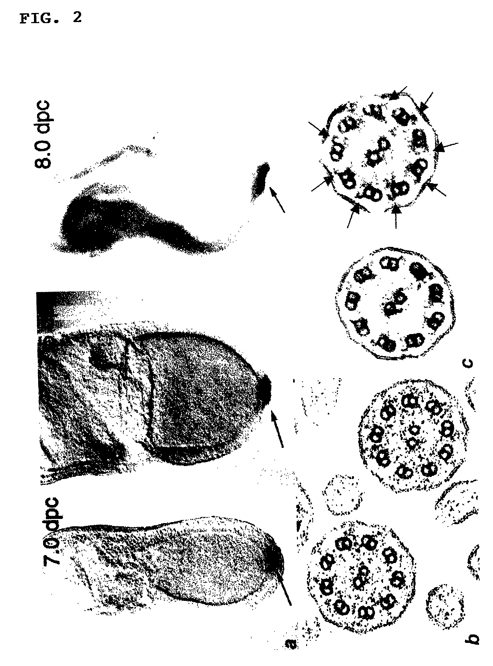

According to the present invention an open reading frame is provided encoding a polypeptide having activity as DNAH5. The nucleotide sequence is shown in Figure 2 and is set forth in SEQ ID No. 1 and the amino acid sequence of DNHA5 is set forth in SEQ ID No. 2.

The present inventors found a composite transcript which harbours an open reading frame of 13 872 nucleotides encoding a protein with 4624 amino acid residues. The DNAH5 genomic region comprises 79 exons and spans 250 kb.

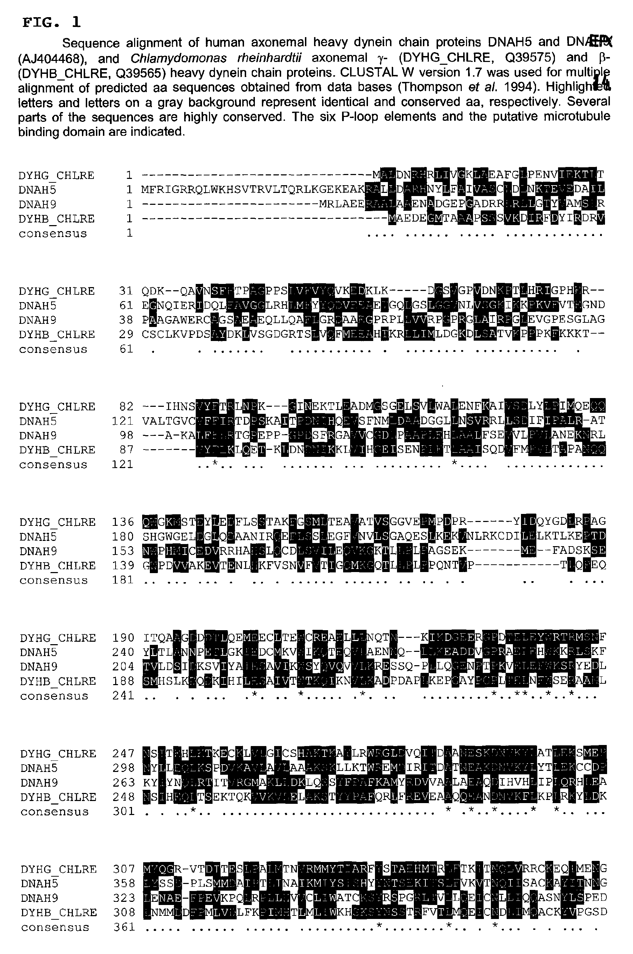

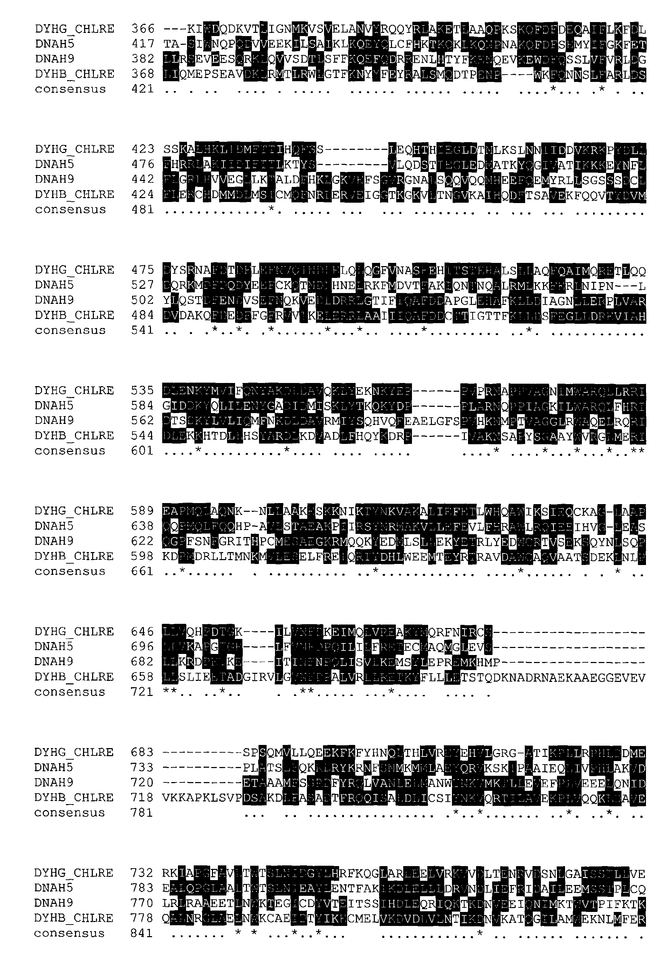

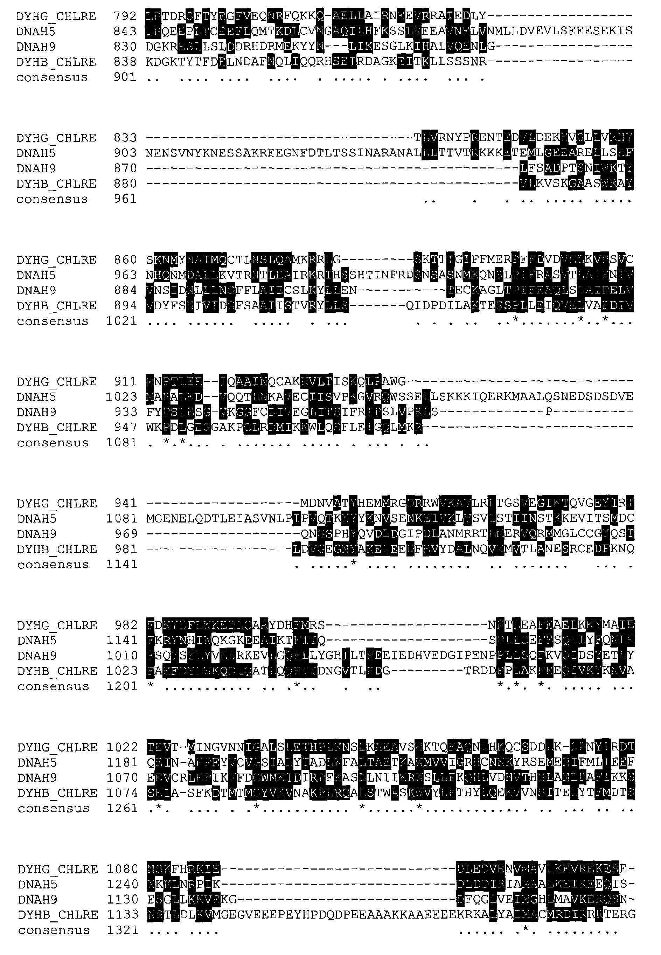

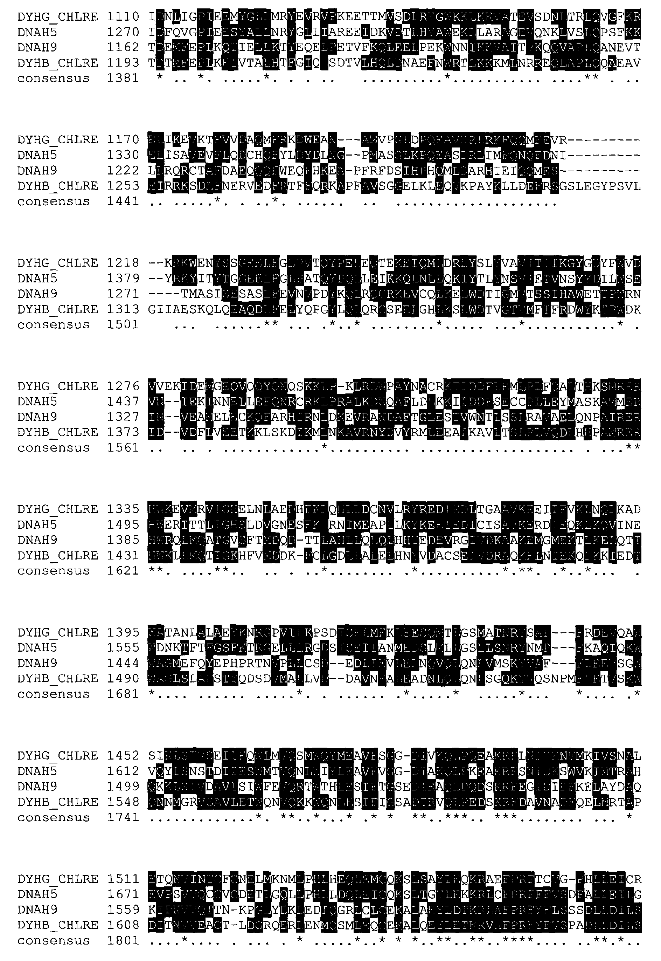

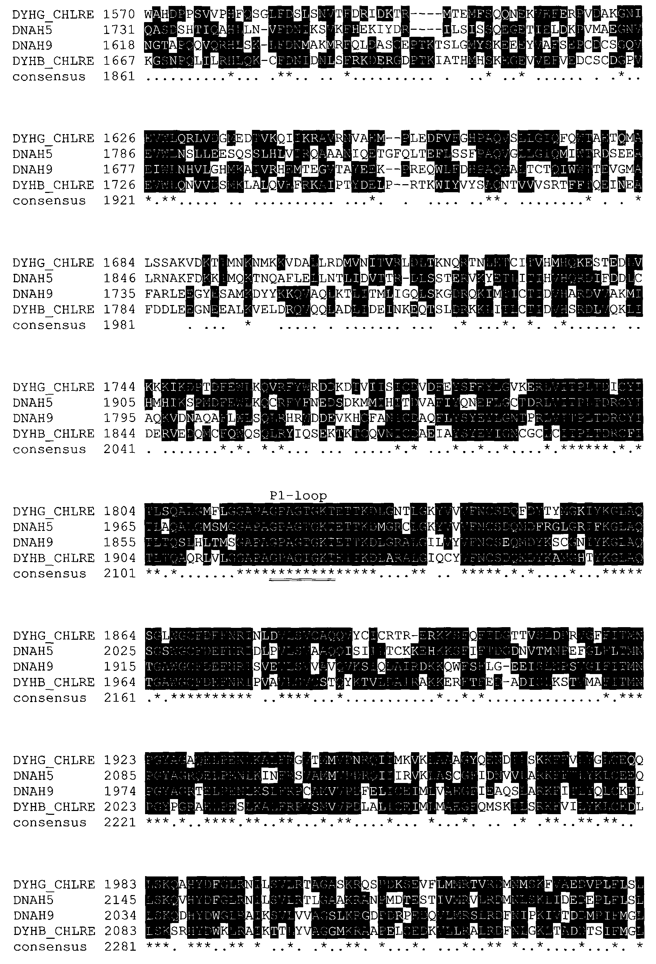

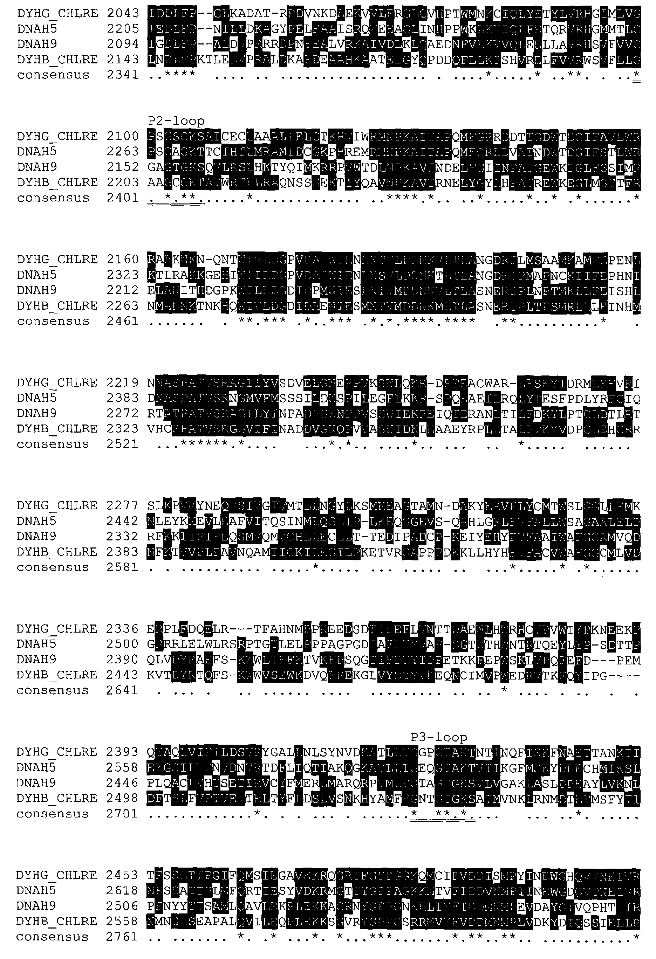

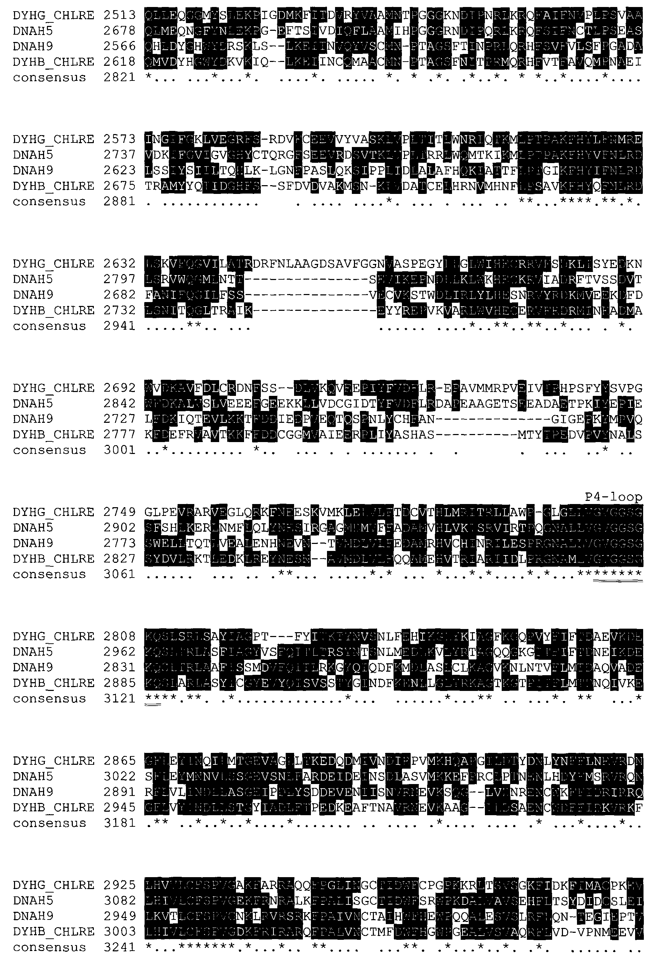

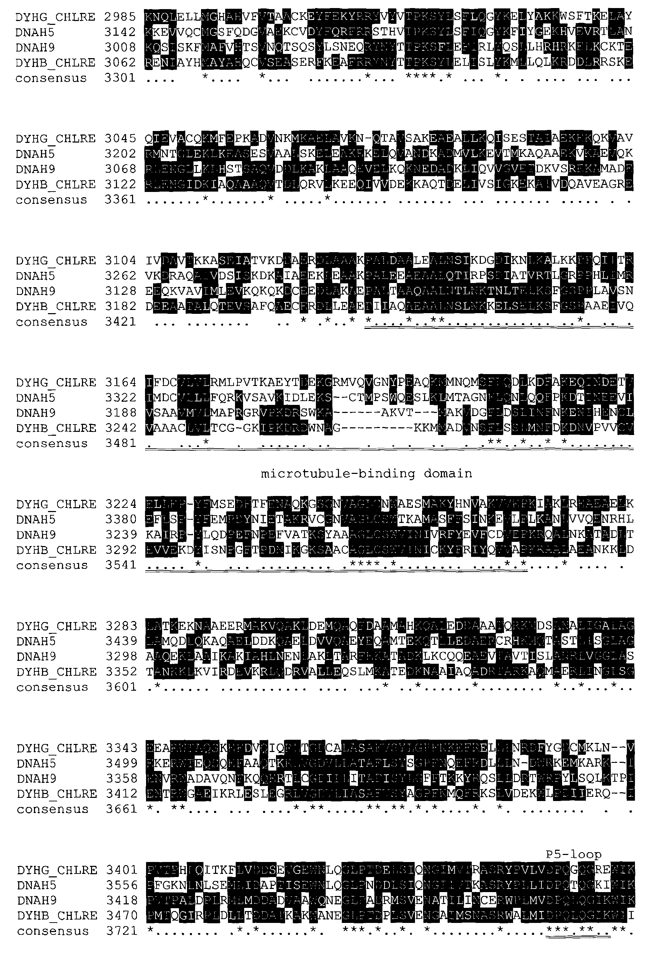

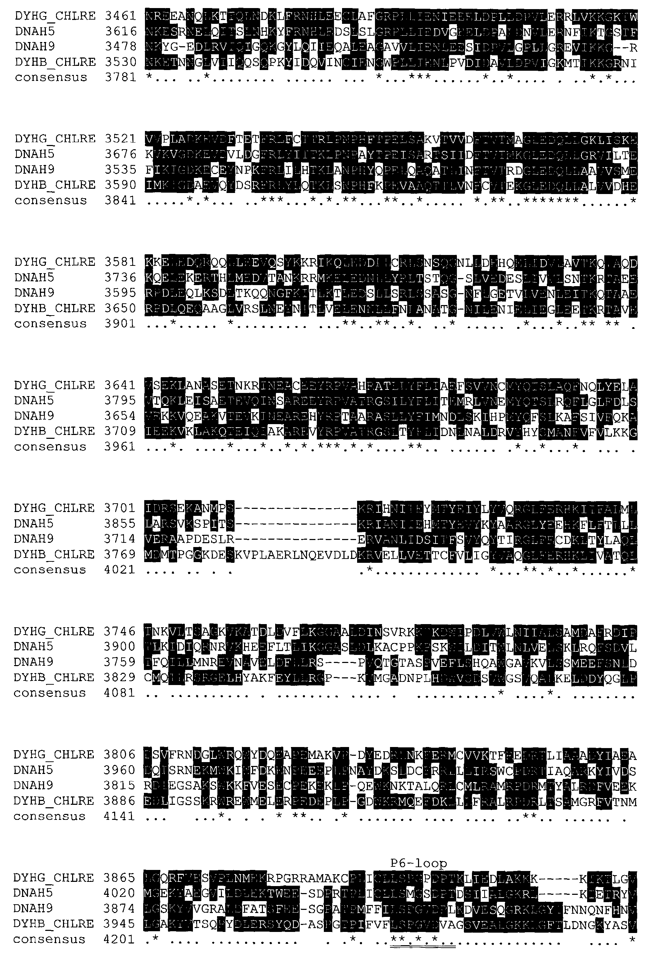

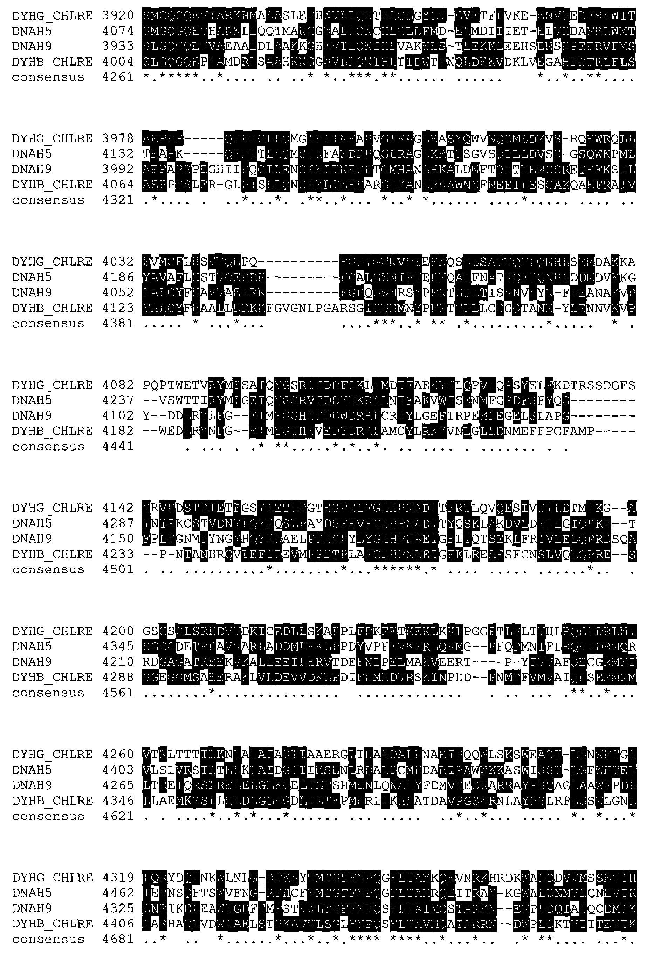

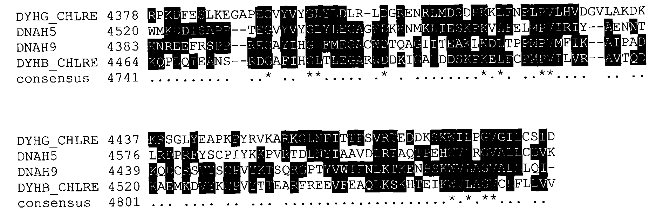

Moreover, an alternative first exon was found by sequencing EST clones which is illustrated in Figure 1.

According to the present invention there is also provided the amino acid sequence of DNAH5 which is set forth in SEQ ID No. 2.

The inventors surprisingly found the dysmotility of cilia is related to defects in the DNAH5 gene or to a failure to produce DNAH5 correctly. Moreover, the inventors found that a disease known as primary ciliary dyskinesia (PCD) manifesting with recurrent infections of the respiratory tract due to reduced mucociliary clearance, sperm dysmotility and situs inversus (reversed organs) is related to DNAH5.

In a further embodiment the present invention provides a method for the diagnosis of diseases which are related to a dysmotility of cilia like primary ciliary dyskinesia, where cilia in the respiratory tract are affected, and for male infertility where the motility of sperms is affected by the defective sperm tail.

The diagnosis can be either done by a mutational analysis where for example RFLPs are determined. The skilled artisan is aware of methods for mutatational analysis.

In another embodiment of the present invention the diagnosis is carried out by using antibodies specific for DNAH5. In the latter case antibodies are raised against DNAH5 or an immunoreactive part or epitope thereof and are used as diagnostic tool. The antibodies can be monoclonal or polyclonal. It is also possible to use immuno-reactive fragments and derivatives thereof, as is known in the art.

The term "immuno-reactivity" or "immuno-reactive" refers to the ability of antibodies and fragments to bind to particular regions (epitopes) presented by the polypeptide or protein. The terms proteins and polypeptide are used interchangably in the present description.

Preferably antibodies are used which have a high binding affinity for the epitope or immuno-active part of the protein. Moreover, it is preferable to use those antibodies which have specific immunoactivity DANH5 or imunoactive parts thereof and essentially do not bind to other proteins.

According to a further aspect of the present invention a method for the diagnosis of cilia dysmotility is provided wherein it is determined if there is a mutation in the DNA sequence of the gene encoding DNAH5. In another embodiment the diagnosis can be done by determining the presence or absence of DNAH5 in a cell of an afflicted patient.

A mutation in the DNA or the presence of defective DNAH5 can establish the diagnosis of a defect of the outer dynein arm. This improves the diagnosis of respiratory problems or the diagnosis of male sterility.

If a defect of the gene coding for DNAH5 has been determined, it is possible to use an isolated and purified protein according to SED ID No. 2 or parts thereof having DNAH5 activity to compensate for the defect.

Moreover, according to another aspect of the present invention a gene therapy can be carried out wherein a nucleotide sequence comprising a gene encoding DNAH5 in functional combination with regulatory sequences is introduced into a cell which then will express DNAH5. It is preferred to use a vector comprising a nucleotide sequence coding DNAH5 together with regulatory sequences which provides for expression of DNAH5 in this cell.

In another aspect of the present invention a biological motor in nano-size is provided. As outlined above dynein is a molecule which acts as a biological motor. Therefore, the DNAH5 sequence or at least the globular motor domain thereof can be used to construct biological motors which are moving in a defined manner.

In a preferred embodiment of the present invention the method for diagnosis of diseases related to a defect of the DNH5 gene is used in the diagnosis of primary ciliary dyskinesia (PCD, MIM 242650). As outlined above PCD is characterized by recurrent infections of the respiratory tract. Therefore, it is favourable to be able to determine if cilia defects are the cause of unclear respiratory problems.

Sequence analysis in PCD patients with randomization of LR asymmetry identified mutations resulting in non-functional DNAH5 proteins. Thus, sequence analysis in patients with unclear respiratory problems or unclear infertility can provide evidence. Previously a PCD locus was localized to chromosome 5p, containing *DNAH5* encoding a protein highly similar to the *Chlamydomonas* γ-dynein heavy chain (DHC)(Omran, H. et al. Am. J. Resp. Cell Mol. Biol. 23, 669-702 (2000). Now, the full-length 14-kb transcript of *DNAH5* has been characterized and is one aspect of the present invention.

Reference is also made to the drawings wherein
Figure 1 shows the Sequence alignment of human axonemal heavy dynein chain proteins DNAH5 and DNAH9 (AJ404468), and *Chlamydomonas rheinhardtii* axonemal - (DYHG_CHLRE, Q39575) and _-(DYHB_CHLRE, Q39565) heavy dynein chain proteins. CLUSTAL W version 1.7 was used for multiple alignment of predicted aa sequences obtained from data bases (Thompson et al. 1994). Highlighted letters and letters on a gray background represent identical and conserved aa, respectively. Several parts of the sequences are highly conserved. The six P-loop elements and the putative microtubule binding domain are indicated.
Figure 2 shows early embryonic expression of murine *Dnahc5* and axonemal phenotype resulting from *DNAH5* mutations. ***a***, Whole mount *in situ* hybridization analysis of murine *Dnahc5* orthologous to human *DNAH5* in early gastrulation stage mouse embryos. *Dnahc5* expression is restricted to the node (arrow) in 7.0 to 8.0 dpc embryos. ***b***, Electron micrograph of cross-sections of respiratory cilia (original magnification x 88,000) from an affected individual of PCD-family (F658). Absence of outer dynein arms is observed on all peripheral doublets. ***c***, Electron micrograph of cross-sections of respiratory cilia (original magnification x 154.000) from a healthy individual for comparison. Location of outer dynein arms are indicated by arrows in the right axoneme.

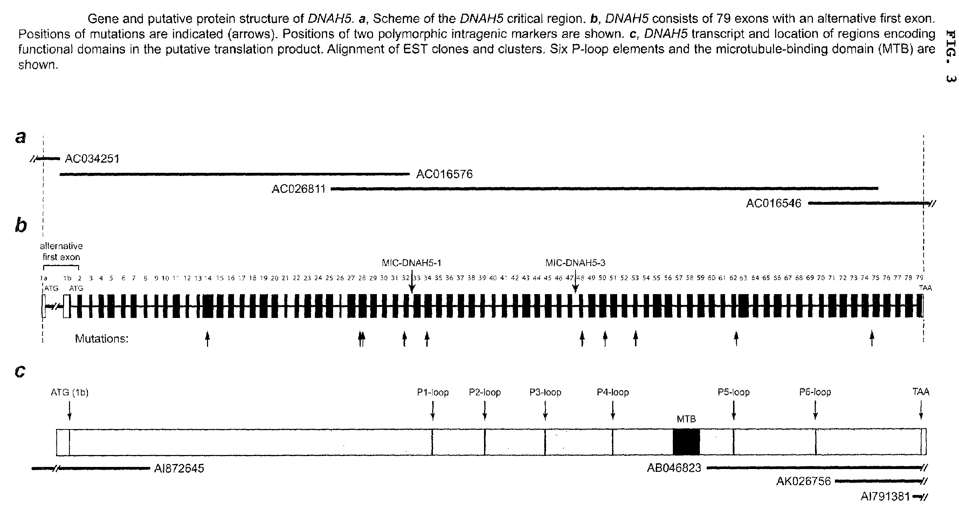

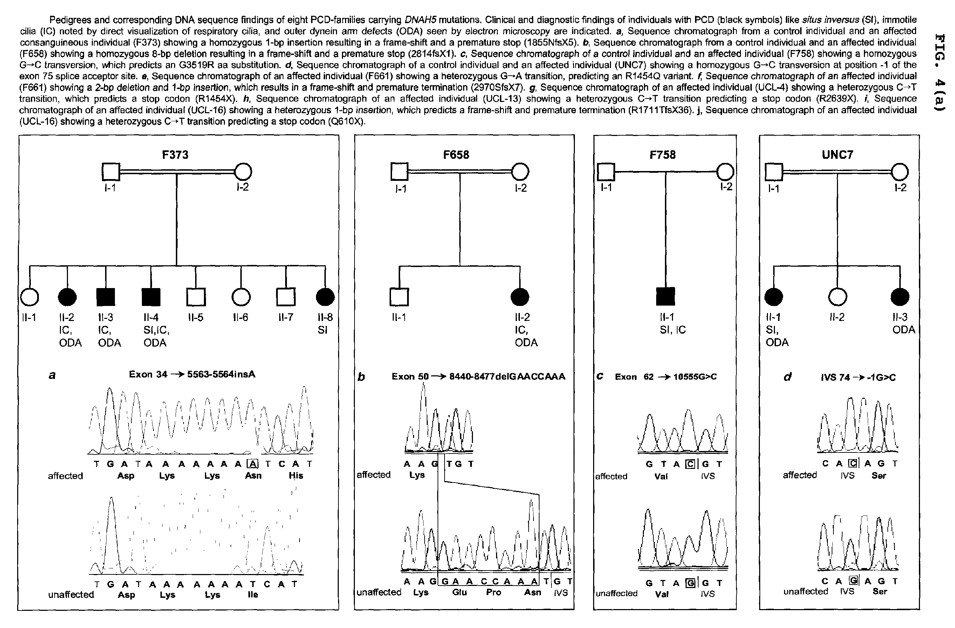

Figure 3 shows Gene and putative protein structure of *DNAH5*. a, Scheme of the *DNAH5* critical region. b, *DNAH5* consists of 79 exons with an alternative first exon. Positions of mutations are indicated (arrows). Positions of two polymorphic intragenic markers are shown. c, *DNAH5* transcript and location of regions encoding functional domains in the putative translation product. Alignment of EST clones and clusters. Six P-loop elements and the microtubule-binding domain (MTB) are shown.

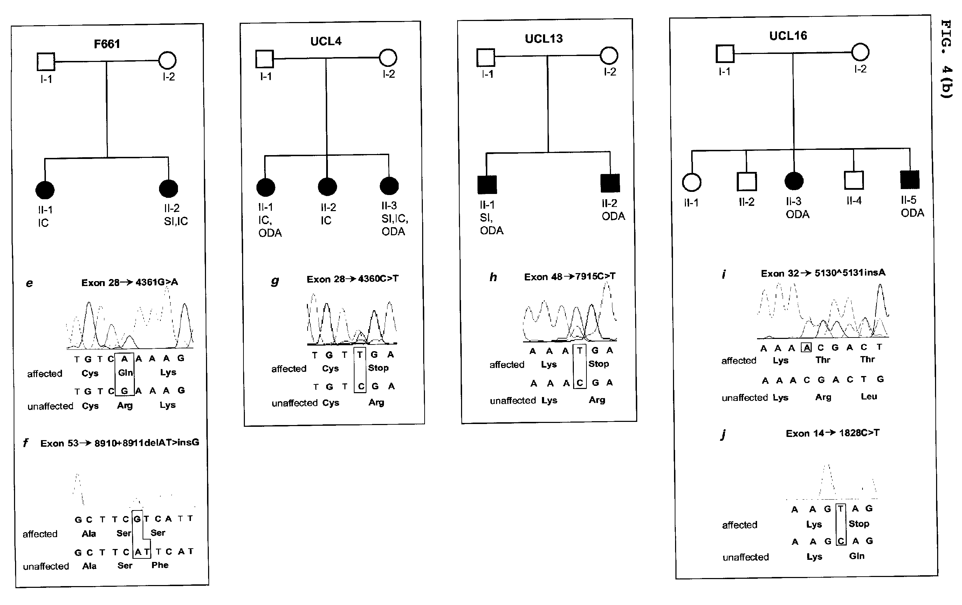

Figure 4 shows Pedigrees and corresponding DNA sequence findings of eight PCD- families carrying DNAH5 mutations. Clinical and diagnostic findings of individuals with PCD (black symbols) like situs inversus (SI), immotile cilia (IC) noted by direct visualization of respiratory cilia, and outer dynein arm defects (ODA) seen by electron microscopy are indicated.
   a) Sequence chromatograph from a control individual and an affected consanguineous individual (F373) showing a homozygous 1- bp insertion resulting in a frame- shift and a premature stop (1855NfsX5).
   b) Sequence chromatograph from a control individual and an affected individual (F658) showing a homozygous 8- bp deletion resulting in a frame- shift and a premature stop (2814fsX1).
   c) Sequence chromatograph of a control individual and an affected individual (F758) showing a homozygous G/C transversion, which predicts an G3519R aa substitution.
   d) Sequence chromatograph of a control individual and an affected individual (UNC7) showing a homozygous G/C transversion at position -1 of the exon 75 splice acceptor site.
   e) Sequence chromatograph of an affected individual (F661) showing a heterozygous G/A transition, predicting an R1454Q variant.
   f) Sequence chromatograph of an affected individual (F661) showing a 2- bp deletion and 1- bp insertion, which results in a frame- shift and premature termination (2970SfsX7).
   g) Sequence chromatograph of an affected individual (UCL- 4) showing a heterozygous C/T transition, which predicts a stop codon (R1454X).
   h) Sequence chromatograph of an affected individual (UCL- 13) showing a heterozygous C Õ T transition predicting a stop codon (R2639X).
   i) Sequence chromatograph of an affected individual (UCL- 16) showing a heterozygous 1- bp insertion, which predicts a frame- shift and premature termination (R1711TfsX36).
   j) Sequence chromatograph of an affected individual (UCL- 16) showing a heterozygous C Õ T transition predicting a stop codon (Q610X).

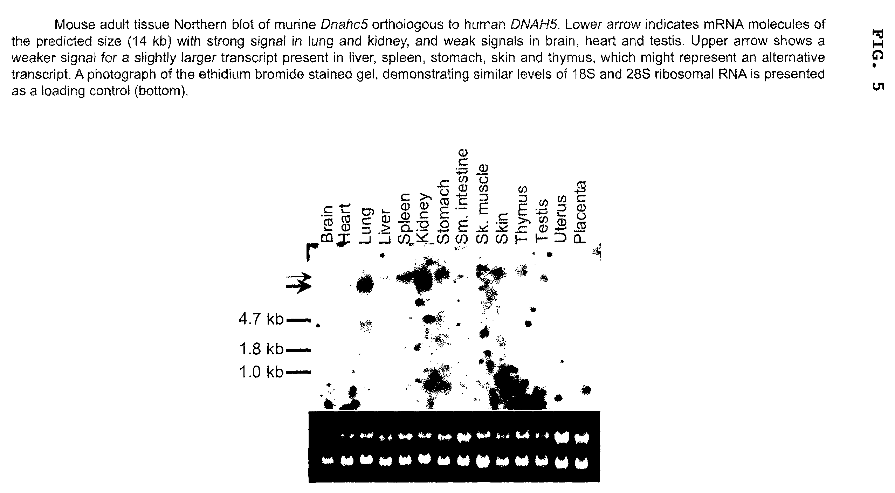

Figure 5 shows Mouse adult tissue Northern blot of murine *Dnahc5* orthologous to human *DNAH5.* Lower arrow indicates mRNA molecules of the predicted size (14 kb) with strong signal in lung and kidney, and weak signals in brain, heart and testis. Upper arrow shows a weaker signal for a slightly larger transcript present in liver, spleen, stomach, skin and thymus, which might represent an alternative transcript. A photograph of the ethidium bromide stained gel, demonstrating similar levels of 18S and 28S ribosomal RNA is presented as a loading control (bottom).

The following experiments and the example are enclosed to illustrate the present invention but are not intended to restrict the scope of the present application.

### Experimental Part

Starting from a 1.5-kb partial cDNA (Omran, H. *et al. Am. J. Resp. Cell Mol. Biol*. **23,** 669-702 (2000), a full-length cDNA for *DNAH5* was obtained by PCR amplification of overlapping cDNA fragments from testis and trachea libraries, as well as from RACE experiments. The composite transcript was found to harbor an open reading frame of 13,872 nucleotides encoding a protein with 4,624 amino acid residues which is shown as SEQ ID No. 1. The *DNAH5* genomic region comprises 79 exons, and spans 250 kb. Evidence was found for an alternative first exon by sequencing of EST clones (See Figure 3).

Northern-blot analysis with a partial cDNA probe of the murine orthologous gene, *Dnahc5* (sequence identity of 91% to *DNAH5*) showed one strong band in lung and kidney, and a weaker signal of the same size in brain, heart, and testis, most likely corresponding to a 14-kb transcript (Fig. 3). To determine embryonic expression of *Dnahc5 in situ* hybridization analysis on whole mouse embryos from 6.5 days *post coitum* (dpc) to 9.5 dpc was carried out. Expression was weak but clearly confined to the node from 7.0 to 8.25 dpc. (Fig. 2a). Pedigrees of all studied PCD-families were compatible with autosomal recessive inheritance. Using haplotype analyses with two intragenic polymorphic markers 25 PCD-families with allele segregation compatible with linkage to *DNAH5* were identified. Four families (F373, F658, F758, and UNC7) showed haplotypes suggesting homozygosity by descent. We searched for mutations by sequencing all exons of *DNAH5* in affected individuals of these 25 PCD-families. 4 homozygous and 6 heterozygous mutations were identified as can be seen from the following table 1 and from Figure 4.

**Table 1**

| Mutations in *DNAH5* | | | | |
|---|---|---|---|---|
| Family | Origin | Exon | DNA change^{a} | Protein change |
| 373 | LB* | [34] + [34] | [5563^5564insA] | [1855NfsX5] |
| 658 | DE | [50] + [50] | [8440-8447delGAACCAAA] | [2814fsX1] |
| 661 | DE | [28] + [53] | [4361G>A]^{b} + [8910+8911delAT>insG] | [R1454Q] + [2970SfsX7] |
| 758 | DE | [62] + [62] | [10555G>C]^{b} | [G3519R] |
| UNC-7 | USA | [75] + [75] | [IVS74-1G>C] | splice-site mutation |
| UCL-4 | GB | [28] + [?] | [4360C>T] + [?] | [R1454X] + [?] |
| UCL-13 | GB | [48] + [?] | [7915C>T] + [?] | [R2639X] + [?] |
| UCL-16 | GB | [14] + [32] | [1828C>T] + [5130^5131insA] | [Q610X] +[R1711TfsX36] |

| | | | | |
|---|---|---|---|---|
| ^{a}Base 1 corresponds to the first base of the initiation codon in the cDNA sequence. | | | | |
| ^{b}Not found in 200 control chromosomes. [?] unknown change | | | | |
| * LB = Lebanon | | | | |

Moreover, mutations on two alleles explaining the recessive phenotype in six PCD families were identified. In two families only one mutation was detected, so that the mutation on the 2^{nd} allele remained to be identified. The cosegregation of the mutations with disease haplotypes by sequencing other available family members was confirmed. Most mutations resulted in premature termination, predicting total or partial loss of the motor and the microtubule binding domain (Fig. 3). Only two missense mutations within conserved domains were found. The eight PCD-families carrying *DNAH5* mutations contained 17 affected individuals. Seven of these affected individuals had Kartagener syndrome, which is defined by an associated complete *situs inversus*¹. The occurrence of affected individuals with and without associated situs inversus within the same PCD family (F373, UNC7, F661, UCL4 and UCL13) indicates randomization of LR asymmetry in PCD patients carrying *DNAH5* mutations (Fig. 4). Ultrastructural studies of respiratory cilia were performed in six families and showed defects of the outer dynein arms (ODA-phenotype) (Fig. 2b,c, Fig. 4). In five PCD-families immotile cilia were noted by direct visualization of respiratory cilia (Fig. 4). These results demonstrate that mutations of *DNAH5* cause PCD with randomization of LR asymmetry. This conclusion is supported by the following findings.

First, *DNAH5* maps to the PCD locus on chromosome 5p². Second, expression of *Dnahc5* is present in lung and the node, explaining the disease phenotype. In addition, four homozygous mutations (including the consanguineous family used to map the locus) and six heterozygous mutations were found in affected individuals of eight PCD-families. Moreover, mutations in the *Chlamydomonas* orthologue are suspected to result in slow swimming algae with a phenotype highly similar on the ultrastructural level³. Thus it appears that the functional role of this gene is evolutionary conserved. This notion is also supported by the high degree of sequence conservation (40% identity, 59% similarity) between both proteins (Fig. 1). The N-terminal third of the protein forms a relatively short tail, which is important for binding of dynein light and intermediate chains⁴. Mutations in *DNAI1* and the orthologous gene of *Chlamydomonas,* which encode intermediate chains also result in PCD or Kartagener syndrome with an ODA-phenotype⁵⁻⁷. This indicates the importance of DNAI1 and DNAH5 for the function of the outer arm dynein complex. The remaining >3000 residues of DNAH5 form the globular motor domain. The dynein motor is organized into six AAA modules^{4,8} and a microtubule-binding domain, which is predicted to form an extended hair-pin like stalk of anti-parallel coiled-coils⁹ (Fig. 3).

Recent studies have shown that monocilia of nodal cells produce an overall leftward flow of extracellular fluid^{10,11}. Disturbances in nodal flow, due to absent nodal cilia in *kif3B*^{-/-}, *kif3A*^{-/-} and *Tg737*^{*Δ2-3βGal*} mice¹⁰⁻¹², and immotile cilia in *lrd*^{*iv*} mutant mice¹³, were associated with randomization of LR patterning. Therefore it has been suggested that the cilia-driven nodal flow creates an asymmetric gradient of a LR morphogen that starts the LR signaling cascade. However, *kif3B*^{-/-}, *kif3A*^{-/-} and *Tg737*^{*Δ2-3βGal*} mice have severe developmental defects resulting in early embryonic death¹⁰⁻¹², arguing that other gene functions might also account for the randomization of LR patterning in these mutant mice. Mutations in *DNAH5*, which cause randomization of LR asymmetry, and expression of *Dnahc5* in the node, give additional support for the nodal flow hypothesis. Interestingly, mutation of *lrd* encoding an axonemal DHC also affects nodal ciliary function¹³.

Based on these results, DNAH5 is the first recognized axonemal DHC, which is essential for nodal and respiratory cilia function. Expression of *DNAH5* in brain and kidney is explained by the presence of ciliated cells in brain lining the ventricles and kidney tubules¹⁴. A potential ciliary function in these organs is supported by *Hfh4* mutants showing hydrocephalus in addition to random LR asymmetry and *Tg737*^{*orpk*} mice developing cystic kidneys and hydrocephalus^{14,15}.
A) Patients and families.
   Signed and informed consent was obtained from each patient and family member using protocols approved by the Institutional Ethics Review Board at the University of Freiburg and collaborating institutions. The diagnosis of PCD was based on i) recurrent- infections of the respiratory tract with development of bronchiectasis with and without situs inversus, and ii) complete lack of ciliary motility by direct visualization of respiratory cilia, and or defects of outer dynein arms on electron microscopic examination in at least one affected family member. Both criteria had to be present in order to meet diagnosis of PCD. The clinical features of the Lebanon family (F373) have previously been reported (Omran, H. *et al. Am. J. Resp. Cell Mol. Biol.* **23,** 669-702 (2000). Only PCD-families with pedigrees compatible with autosomal recessive inheritance were included in the study.
B) Isolation of *DNAH5* and analysis of the protein sequence. Starting from a partial cDNA sequence (Omran et al. 2000, AY049075), BLAST searches (Altschul S. F. *et al. Nucleic. Acids. Res.* **25,** 3389-3402 (1997) were carried out with the TBLASTN algorithm against the htgs data base, which revealed that *DNAH5* resides on BAC clones AC016576, AC026811 and AC016546. GenScan predictions (http://genome.dkfz-heidelberg.de/cgi-bin/GENSCAN/ genscan.cgi) were carried out with parts of the clones showing homology to heavy dynein chains. Conformation of computer predictions and filling of sequence gaps was achieved by amplification of overlapping fragments of testis cDNA and subsequent direct sequencing of both strands. The missing 5'- and 3'- ends were obtained by RACE-PCR on testis and trachea cDNA (Marathon Ready, Clontech). An alternative 5' end was identified by sequencing human EST clone AI872645. The CLUSTAL W algorithm, NEWCOILS program, BLASTP/N/X and TWOBLAST were used for sequence analysis (Altschul *et al*. 1997, Thompson, J.D., Higgins, D.G. & Gibson, T.J. *Nucleic Acids Res.* **22,** 4673-4680 (1994).
C) Generation of simple tandem repeat polymorphisms from *DNAH5* region and genotyping.
   BAC clones AC016576 and AC026811 were searched for intragenic simple tandem repeat sequences by alignment of 20-bp sequences of di-, tri- and tetranucleotide repeat elements using TWOBLAST (Altschul et al. 1997). Then the following primer pairs were selected using the Primer3 program (http://www-genome.wi.mit.edu/cgi-bin/primer/primer3_www.cgi): M IC-DNAH5-1a ( 5'-TTTCCCCCCTTACAGTATGG- 3') (SEQ ID No. 3) and MIC-DNAH5-1b (5'-AACAAGAGCGAGACTCCGTC- 3') (196-bp product, intron 32) (SSEQ ID No. 4); MIC-DNAH5-3a (5'-TAGCCAACATTGCACTCCAG- 3') (SEQ ID No. 5) and M IC-DNAH5-3b ( 5'-CTGTCCTTGAGAATCTTCACA- 3') (161-bp product, intron 47) (SEQ ID No. 6). Microsatellite markers were amplified by PCR and analyzed on polyacrylamide gels following described methods (Omran, H., Haeffner, K., Burth, S., Ala-Mello, S., Antignac, C. & Hildebrandt, F. *Nephrol. Dial. Transplant.* **16,** 755-758 (2001). Heterozygosity indices observed in studied families for MIC-DNAH-5-1 and MIC-DNAH5-3 were 0,87 and 0,62, respectively.
D) Detection of *DNAH5* mutations.
   A mutational analysis in affected individuals of 25 informative PCD-families was performed with haplotype analyses compatible with linkage to *DNAH5*. 80 genomic fragments containing all 79 exons and one alternative first exon of *DNAH5* were amplified. Mutations were detected by direct sequencing of PCR amplification products. All mutations were verified bidirectionally. Segregation analyses and screening of controls for mutations was done by direct sequencing or allele-specific digests.
E) Northern-blot analysis.
   Expression and transcript size of the mouse orthologue *Dnahc5* was determined by northern-blot analysis using blots containing poly (A)+ RNA isolated from different tissues (MATB 1006-1, Seegene). The blot was hybridized with a 1.1 kb *Dnahc5* cDNA probe (AA880561) random labeled with 32 P-dCTP (N5000, Amersham Pharmacia Biotech). Hybridization and autoradiography were performed according to manufacturer' s instructions.
F) *In situ* hybridization analysis.
   Whole-mount *in situ* hybridization on mouse 6.5 dpc to 9.5 dpc. embryos using a digoxigenin labeled antisense riboprobe transcribed from a 1.1 kb mouse cDNA (AA880561) was performed as described following a published modification (Parr, B.A., Shea, M.J., Vassileva, G. & McMahon, A.P. *Development* **119,** 247-261 (1993); Knecht, A.K., Good, P.J., Dawid, I.B. & Harland, R.M. *Development* **121,** 1927-1936 (1995). Color reactions were extended up to 4 d to visualize the weak expression in the node. Whole-mount specimen were transferred into 80% glycerol and photographed using Leica DC200 digital camera on a Leica M420 photomicroscope or under Nomarski optic using a Fujix digital camera HC300Z on a Zeiss Axioplan. Files were processed in Adobe Photoshop v.5.0 on G4 PowerMacintosh.
G) GenBank accession numbers.
   Human *DNAH5* nucleotide sequence (AY045575). Partial murine *Dnahc5* nucleotide sequence (AA880561).

### References

**1.** Afzelius, B.A. & Mossberg, B. *The Metabolic and molecular bases of inherited disease* (eds. Scriver, C.R., Beaudet, A.L. & Sly, W.S.) 3943-3954 (McGraw-Hill, New York, 1995).
**2.** Omran, H. et al. Am. J. Resp. Cell Mol. Biol. **23,** 669-702 (2000).
**3.** Wilkerson, C.G., King, S.M. & Witman, G.B. *J. Cell Sci.* 107, 497-506 (1994).
**4.** Asai J. & Koonce M.P. *Trends Cell Biol.* **11,** 196-202 (2001).
**5.** Pennarun, G. *et al. Am. J. Hum. Genet*. **65,** 1508-1519 (1999).
**6.** Guichard, C. *et al. Am. J. Hum. Genet.* **68,** 1030-1035 (2001).
**7.** Wilkerson, C.G., King, S.M., Koutoulis, A., Pazour, G.J. & Witman, G.B. *J. Cell Biol*. **129,** 169-178 (1995).
**8.** Mocz, G. & Gibbons, R. *Structure* **9,** 93-103 (2001).
**9.** Gee, M.A., Heuser, J.E. & Vallee, R.B. *Nature* **390,** 636-639 (1997).
**10.** Nonaka, S. *et al. Cell* **95,** 829-837 (1998).
**11.** Takeda, S. *et al. J. Cell Biol.* **145,** 825-836 (1999).
**12.** Murcia, N.S. *et al. Development* **127,** 2347-2355 (2000).
**13.** Supp, D.M., Witte, D.P., Potter, S.S. & Brueckner M. *Nature* **389,** 963-966 (1997).
**14.** Taulman, P.D., Haycraft, C.J., Balkovetz, D.F. & Yoder, B.K. *Mol. Biol. Cell* **12,** 589-599 (2001).
**15.** Chen, J., Knowles, H.J., Hebert, J.L. & Hackett, B.P. *J. Clin. Invest.* **102,** 1077-1082 (1998).

## Claims

1. A DNA comprising the open reading frame of the gene for DNAH5.

2. A DNA comprising a or a nucleotide sequence complementary thereto which hybridizes under stringent conditions to the nucleotide sequence set forth in SEQ ID No. 1.

3. The DNA of claim 2 comprising at least nucleotides 43 to 1915 of SEQ ID No. 1.

4. Vector comprising the nucleotide sequence of one of claims 1 to 3 and regulatory sequences necessary for the replication and/or expression thereof.

5. An isolated and purified polypeptide having the amino acid sequence of DNAH5.

6. An isolated and purified polypeptide having the amino acid sequence according to SEQ ID No. 2 or an active part thereof, or an amide thereof, or an ester thereof, or a salt thereof.

7. The polypeptide of claim 6, wherein the active part is an amino acid sequence which has the activity of DNAH5 and/or binds to DNAH5 specific antibodies.

8. Purified partial peptide comprising an epitope bearing portion of the polypeptide of one of claims 4 to 7.

9. DNA having a nucleotide sequence as set forth in one of SEQ ID No. 3, 4, 5 or 6.

10. An antibody against the polypeptide of one of claims 3 to 7 or an amide thereof, or an ester thereof, or a salt thereof.

11. Antibody according to claim 10 wherein the antibody is a monoclonal or polyclonal antibody, or a DNAH5 binding fragment or derivative thereof.

12. A method of detecting cilia dysmotility by detecting a mutation in the DNAH5 gene of an afflicted person.

13. The method according to claim 12 wherein tandem repeat polymorphisms are used for the detection.

14. The method for the diagnosis of a disease which is related to cilia dysmotility by detecting the presence or absence of DNAH5 in cells of the afflicted person.

15. A method for the diagnosis of male infertility or sterility wherein a genetic analysis of the DNAH5 gene in an afflicted person is carried out.

16. Use of antibodies against DNAH5 for diagnosis PCD, male infertility or sterility, ciliary and flagellar defects of the outer dynein arms.

17. A method for quantitating DNAH5, or a salt thereof, or an amide thereof, or an ester thereof which comprises using the antibody according to claim 10.

18. Use of the DNA of claim 1 or claim 2 or the vector of claim 3 to introduce the DNAH5 gene in a human cell having a defect in the said gene to express or increase the level of expression of the DNAH5 gene.

19. Use of claim 18 wherein the human cell is a testis, trachea, kidney or lung cell.

20. Use of claim 18 for prenatal diagnosis.

21. A composition for the diagnosis of cilia dysmotility, PCD, male infertility or sterility, or a ciliary or flagella defect comprising antibodies according to claim 10 or 11.

22. A composition for treatment of PCD comprising DNAH5 according to one of claims 5 to 7.

23. Nucleotide sequence encoding a biological motor comprising at least those nucleotides of SEQ ID No. 1 coding for the globular motor domain.

24. Nano-sized biological motor comprising at least the globular motor domain of DNAH5.
